# FASCICULE DE BREVET EUROPEEN

(11) **EP 2 810 075 B1**
(45) Date de publication et mention de la délivrance du brevet: **16.10.2019**
(21) Numéro de dépôt: 13702062.4
(22) Date de dépôt: 01.02.2013
(51) Int. Cl.: G01N 33/543, G01N 33/564, G01N 33/68

(54) **PUCES A PROTEINES, PREPARATION ET UTILISATIONS**
PROTEINCHIPS, HERSTELLUNG UND VERWENDUNG DAVON
PROTEIN CHIPS, PREPARATION AND USE THEREOF

(30) Priorité: 01.02.2012 FR 1250956
(43) Date de publication de la demande: 10.12.2014
(73) Titulaire: Centre National de la Recherche Scientifique (C.N.R.S.), 75016 Paris (FR); Université René Descartes Paris 5, 72270 Paris Cedex 06 (FR); Les Hospices Civils de Lyon, 69424 Lyon Cedex 03 (FR); Université Claude Bernard Lyon 1, 69100 Villeurbanne (FR)
(72) Inventeur: LESAGE, Florian, F-06560 Valbonne (FR); CHATELAIN, Franck, F-06600 Antibes (FR); MAZZUCA, Michel, F-75018 Paris (FR); ROGEMOND, Véronique, F-69003 Lyon (FR); LARROQUE, Marie-Madeleine, F-06600 Antibes (FR); HONNORAT, Jérome, F-69500 Bron (FR)
(74) Mandataire: LLR
(86) Numéro de dépôt international: PCT/EP2013/052009
(87) Numéro de publication internationale: WO 2013/113864

(56) Documents cités:
- WO-A1-2004/108762
- WO-A1-2011/142900
- WO-A2-2006/033972
- IRANI S ET AL: "Autoantibody-mediated disorders of the central nervous system", AUTOIMMUNITY 200802 GB LNKD- DOI:10.1080/08916930701619490, vol. 41, no. 1, février 2008 (2008-02), pages 55-65, XP008154131, ISSN: 0891-6934 cité dans la demande
- H. PRÜSS ET AL: "Anti-NMDA-receptor encephalitis", DER NERVENARZT, vol. 81, no. 4, 31 janvier 2010 (2010-01-31), pages 396-408, XP055034322, ISSN: 0028-2804, DOI: 10.1007/s00115-009-2908-9 cité dans la demande
- ROBINSON W H ET AL: "Protein and Peptide Array Analysis of Autoimmune Disease", BIOTECHNIQUES, INFORMA HEALTHCARE, US, no. SUPPL, 1 décembre 2002 (2002-12-01), pages 66-69, XP001525556, ISSN: 0736-6205
- Anonymous: "Protein Analysis - Access the human proteome on a microarray scale - ProtoArray(R) Human Protein Microarray v5.0", , 7 June 2011 (2011-06-07), XP055456462, Retrieved from the Internet: URL:https://www.thermofisher.com/content/d am/LifeTech/migration/en/filelibrary/prote in-expression/pdfs.par.70966.file.dat/prot oarray%20v5%20general-fhr.pdf [retrieved on 2018-03-05]
- Anonymous: "Absolute identification of novel autoimmune biomarkers ProtoArray (R) Human Protein Microarrays", , 7 June 2011 (2011-06-07), XP055254290, Retrieved from the Internet: URL:https://www.thermofisher.com/content/d am/LifeTech/migration/en/filelibrary/prote in-expression/pdfs.par.16180.file.dat/prot oarray%20v5%20irbp-fhr.pdf [retrieved on 2016-03-01]

## Description

La présente invention concerne le domaine de la médecine, en particulier de la recherche et du diagnostic. Elle concerne plus particulièrement un nouvel outil permettant la détection d'autoanticorps au sein d'un échantillon biologique provenant d'un mammifère. Cet outil, se présentant sous la forme d'une puce à protéines, est typiquement utilisable dans le criblage de nouvelles cibles d'intérêt impliquées dans la survenue d'une maladie autoimmune, en particulier d'une maladie affectant le système nerveux d'un mammifère, ainsi que dans le diagnostic ou le suivi de l'évolution d'une telle maladie autoimmune. L'invention concerne également un procédé de fabrication d'un tel outil ainsi que des kits le comprenant et permettant son utilisation.

### ART ANTERIEUR

L'art antérieur propose plusieurs types de tests permettant de détecter la présence d'une molécule cible dans un échantillon. Parmi ceux-ci figurent les radio-immunoessais (RIA) ; les tests d'immunofluorescence (IFA) ; les immunoessais par luminescence (LIA) ; les tests EIA (Enzyme immuno assay), en particulier les tests ELISA (Enzyme-linked immunosorbent assay), littéralement « dosage d'immunoadsorption par enzyme liée », c'est-à-dire dosage immuno-enzymatique sur support solide.
Le RIA (radio-immunoessai) permet de mesurer les concentrations d'antigènes dans un échantillon en utilisant des anticorps marqués par un radioélément. Le dosage du radioélément mesure un nombre de désintégrations par seconde. Cette technique est la technique inverse du RBA (« radiobinding assay ») qui permet de quantifier un anticorps à l'aide des antigènes correspondants. Ces techniques sont simples à mettre en oeuvre et peu coûteuses mais nécessitent l'usage de substances radioactives, souvent des isotopes de l'iode liés à des résidus de tyrosine. Les tests de radioimmunoprécipitation (RIPA) permettent la précipitation d'un antigène d'intérêt présent dans un échantillon à l'aide d'un anticorps spécifique marqué. Ce procédé peut être utilisé pour isoler et concentrer une protéine particulière au sein d'un échantillon en comprenant des milliers d'autres. La technique requiert que l'anticorps soit couplé à un substrat solide.
Les tests d'immunofluorescence (IFA) utilisent des anticorps (ou immunoglobulines) ainsi que des fluorochromes (substance chimique capable d'émettre de la lumière de fluorescence après excitation). Les fluorochromes les plus utilisés sont la phycoérythrine (PE), le Fluorescéine isothiocyanate (FITC), la gamme d'Alexa Fluor, la protéine fluorescente verte (green fluorescent protein, GFP). Ces tests présentent l'avantage de donner des résultats très rapidement, voire immédiatement, en s'affranchissant des longs temps d'exposition requis pour la technique par radioactivité. La fluorescence présente l'inconvénient de ne pas être permanente, l'intensité de la fluorescence diminuant avec le temps jusqu'à devenir indétectable. Dans le cadre des immunoessais par luminescence (LIA), l'activité enzymatique est mesurée par luminométrie.
L'ELISA (Enzyme-linked immunosorbent assay) entre dans le cadre plus général des EIA (enzyme immunoassays) ou dosages immunoenzymatiques dans lesquels le dosage d'une réaction entre antigène et anticorps est couplé à une réaction catalysée par une enzyme qui libère un composant coloré suivi par une spectroscopie. L'ELISA est une technique biochimique, principalement utilisée en immunologie afin de détecter la présence d'un anticorps ou d'un antigène dans un échantillon (e.g. Gotti et al. Muscle & Nerve, 20 : 800-808, 1997 ; Franciotta et al., Clin Chem. 45 : 400-5, 19999 ; Hewer et al., Clin Chim Acta. 364 :159-66, 2006). La technique utilise un ou deux anticorps. L'un de ceux-ci est spécifique de l'antigène, tandis que l'autre réagit aux complexes immuns (antigène-anticorps) et est couplé à une enzyme. Cet anticorps secondaire, responsable du nom de la technique, peut aussi causer l'émission d'un signal par un substrat chromogène ou fluorogène.

Les tests disponibles et décrits ci-dessus ne sont cependant pas assez sensibles pour détecter des concentrations très faibles de molécules cibles, typiquement d'anticorps, en particulier d'autoanticorps et encore plus particulièrement d'autoanticorps dirigés contre des protéines membranaires et/ou complexes protéiques membranaires.
Par ailleurs, ils doivent généralement être mis en oeuvre sur de petites quantités de sérum. Une dilution s'avère ainsi souvent nécessaire pour éviter un bruit de fond excessif. Les tests ELISA par exemple peuvent nécessiter une dilution du sérum d'au moins 50 fois. Les tests de radio-immunoprécipitation (RIPA) privilégient l'utilisation de seulement quelques microlitres (environ 5 microlitres) de sérum à tester afin d'éviter l'utilisation de grands volumes d'anti-sérum générant des bruits de fonds inacceptables (i.e. valeurs inappropriées de la radioactivité des contrôles négatifs) du fait de culots excessivement larges. Même si certains tests ELISA spécifiques peuvent utiliser des volumes de sérum plus grands (e.g. 50 à 100 micro litres), leur utilisation est toutefois limitée et leur sensibilité n'est pas augmentée. Par exemple, un kit commercial ELISA de RSR Ltd pour la détection d'anticorps anti-récepteur nicotinique de l'acétylcholine (AChR) associés à la myasthénie gravis permet l'utilisation d'une combinaison de 3 anticorps monoclonaux anti-AChR dans un essai de type sandwich utilisant 100 microlitres de sérum. Cette technique n'augmente toutefois pas la sensibilité de l'essai par rapport au test RIA standard. En outre, d'après Irani et al. (Autoimmunity, February 2008 ; 41(1) : 55-65) la technique ELISA, tout comme les techniques de type « Western blotting » et « phage display », permet l'identification d'autoanticorps dirigés contre des épitopes non conformationnels mais pas celle des autoanticorps dirigés contre des épitopes fonctionnels. La technique du « Western blotting » de même que l'immunohistochimie impliquent notamment la dénaturation des protéines d'intérêt (H. Prüss et al : « Anti-NMDA-receptor encephalitis », Der Nervenartz, vol. 81, no. 4, 31 janvier 2010, pp. 396-408).

Un certain nombre d'études récentes (Vincent et al., Autoimmune channelopathies and related neurological disorders. Neuron 2006 52:123-138, et Vernino et al., Autoimmune encephalopathies. Neurologist 2007 13(3) :140-7) montrent la présence et l'implication d'autoanticorps dans des pathologies affectant le système nerveux. La notion que les neurones puissent être la cible d'une attaque autoimmune, en particulier dans le système nerveux central, est assez récente. L'article de Graus et al. (J. Antibodies and neuronal autoimmune disorders of the CNS. J. Neurol. 2010 257(4) :509-17) suggère la détection d'autoanticorps pour diagnostiquer des syndromes neurologiques paranéoplasiques. L'article d'Irani et al. (Autoantibody-mediated disorders of the central nervous system. Autoimmunity 2008 41(1) :55-65) décrit l'existence d'autoanticorps dirigés contre les canaux ioniques et les récepteurs de surface ayant des effets pathogènes dans la myasthénie gravis et la neuromyotonie acquise. La pathogénicité de ces anticorps est supportée par le fait que des encéphalites et des syndromes neurologiques paranéoplasiques (SNP) répondent positivement aux traitements immunomodulateurs et aux plasmaphérèses. De plus, cette réponse thérapeutique positive est corrélée à une diminution du taux de ces autoanticorps dosés dans le liquide céphalo-rachidien.
D'un point de vue pratique, la description régulière de nouveaux autoanticorps et le spectre des syndromes associés sont tels que les neurologues ont encore du mal à estimer quel anticorps ou quel ensemble d'anticorps est utile pour diagnostiquer un syndrome donné. L'importance et le potentiel de tels marqueurs pour le diagnostique et le traitement ne font cependant pas de doute et requièrent le développement de méthodes systématiques de détection et de caractérisation de ces anticorps afin de répondre au besoin d'améliorer très sensiblement les corrélations entre syndrome clinique, signature immunologique et réponse thérapeutique.

Parmi les pathologies affectant le système nerveux (désordres ou maladies neurologiques) ne bénéficiant pas à l'heure actuelle de méthode de diagnostique adéquate figurent notamment les encéphalopathies, en particulier celles associées à des troubles de l'excitabilité neuronale.
Il n'existe en effet à l'heure actuelle aucun outil qui soit suffisamment sensible et spécifique pour être en mesure de détecter de manière efficace les autoanticorps, souvent présents en très faibles quantités, dirigés contre des protéines membranaires ou complexes protéiques membranaires, en particulier des protéines ou complexes comprenant des épitopes fonctionnels, décrits comme impliqués, ou suspectés d'être impliqués, dans la survenue de ces encéphalopathies chez l'homme. Ainsi par exemple, la demande de brevet WO2011/142900 qui décrit une méthode de diagnostic d'une maladie neurodégénérative comprenant la détection d'autoanticorps ainsi qu'une puce fabriquée à l'aide de protéines, précise toutefois que ces protéines sont produites dans des cellules d'insecte, et non, contrairement à l'invention, dans des cellules de mammifère, avant d'être purifiées. Une telle méthode de production impacte inévitablement les modifications post-traductionnelles des protéines ainsi produites et donc la reconnaissance d'épitopes par d'éventuels auto-anticorps. Il est en outre impossible à l'aide d'une telle puce de permettre la coexpression à la surface de ladite puce de sous-unités partenaires (complexe protéique). WO 2006/033972 porte sur la préparation de puces à protéines, tous les exemples décrivant l'expression des protéines recombinantes dans des cellules d'insectes. Le procédé décrit dans WO 2006/033972 implique une étape de purification des protéines par chromatographie d'affinité. Les autres type de tests d'identification d'autoanticorps pratiqués à l'heure actuelle nécessitent quant à eux généralement le sacrifice d'animaux, typiquement de rongeurs (rats, souris), dans la mesure où ils sont tous effectués sur des cellules, en particulier sur des cellules transfectées, ou sur des coupes de tissus (typiquement disposées sur des lames de verre).

### RESUME DE L'INVENTION

L'invention a pour objet un procédé de préparation d'une puce à protéine, comprenant les étapes suivantes :
a) clonage de l'ADNc ou des ADNc d'intérêt codant au moins une protéine membranaire s'exprimant dans les cellules du système nerveux d'un animal, impliquée ou suspectée d'être impliquée dans la survenue d'une encéphalopathie associée à des troubles de l'excitabilité neuronale,
b) expression de la ou des protéines membranaires codées par ledit ou lesdits ADNc dans des cellules en culture, lesdites cellules en culture étant des cellules de mammifères, à l'aide d'un vecteur permettant i) l'insertion d'une étiquette (séquence tag) dans le même cadre de lecture que la protéine d'intérêt et ii) l'expression d'une protéine fusionnée à ladite étiquette,
c) solubilisation des protéines fusionnées à ladite étiquette exprimées à l'aide d'un détergent non dénaturant permettant la solubilisation desdites protéines membranaires tout en préservant leur conformation et leur capacité de fixation à un support, pour obtenir un solubilisat et
d) dépôt du solubilisat desdites protéines membranaires obtenu à l'issue de l'étape c) sur un support et obtention de la puce d'intérêt.
Les inventeurs décrivent à présent, une puce à protéines permettant la détection d'anticorps (en particulier l'identification de nouveaux anticorps), de préférence d'autoanticorps, encore plus préférentiellement d'autoanticorps dirigés contre des protéines membranaires et/ou complexes protéiques membranaires, dans un échantillon biologique. Cette puce comprend au moins une protéine d'intérêt solubilisée et se présente sous la forme d'un support permettant la détection des complexes formés entre anticorps, de préférence autoanticorps, et épitopes spécifiques de ladite protéine et/ou dudit complexe protéique, lesdits épitopes spécifiques pouvant être des épitopes séquentiels ou conformationnels. Ladite au moins une protéine d'intérêt solubilisée est une protéine membranaire d'intérêt solubilisée et la puce à protéines et une puce à protéines membranaires. La protéine membranaire d'intérêt solubilisée s'exprime dans les cellules du système nerveux d'un animal, de préférence d'un être humain, et est impliquée ou suspectée d'être impliquée dans la survenue d'une encéphalopathie associée à des troubles de l'excitabilité neuronale.

Un avantage considérable de la puce à protéines selon l'invention est de permettre, du fait de la préservation par la protéine de sa structure tridimensionnelle native, la détection à la fois sensible et spécifique de tout anticorps, présent dans un échantillon biologique, capable de lier l'un quelconque des épitopes (séquentiels aussi bien que conformationnels) formés par la ou les protéines d'intérêt exprimées sur ladite puce. La protéine membranaire d'intérêt peut être seule (protéine membranaire) ou associée à une ou à plusieurs sous-unités auxiliaires membranaires ou périmembranaires (complexe protéique membranaire). De par sa spécificité, cette puce permet par ailleurs l'utilisation de grandes quantités d'échantillons biologiques à tester et n'impose, dans le cadre de son utilisation, aucune étape de dilution dudit échantillon biologique. Il est ainsi désormais possible de détecter la présence d'anticorps même lorsque leur concentration dans l'échantillon à tester est très faible.

Un objet particulier de l'invention concerne une puce à protéines permettant l'identification simple et efficace d'anticorps, de préférence d'autoanticorps, dans un échantillon biologique, ladite puce comprenant au moins une protéine d'intérêt solubilisée, typiquement au moins une protéine membranaire d'intérêt solubilisée de préférence choisie parmi une protéine canal ionique, une protéine transporteur, et une protéine récepteur membranaire régulant l'activité d'une protéine canal ionique ou d'une protéine transporteur, ladite protéine étant éventuellement associée à une ou plusieurs sous-unités auxiliaires, et ladite puce se présentant sous la forme d'un support permettant une détection des complexes formés entre anticorps, de préférence autoanticorps, et épitopes (séquentiels aussi bien que conformationnels) spécifiques de ladite protéine, éventuellement associée à sa ou à ses sous-unités auxiliaires. De manière préférée ladite au moins une protéine ou ledit au moins un complexe protéique membranaire s'exprime dans le système nerveux d'un être humain et est, de préférence, impliquée ou suspectée d'être impliquée dans la survenue d'une encéphalopathie associée à des troubles de l'excitabilité neuronale.

Un autre objet de l'invention concerne une puce à protéines comprenant plusieurs protéines d'intérêt différentes, typiquement plusieurs protéines membranaires d'intérêt différentes, typiquement au moins 50 ou au moins 100 protéines d'intérêt différentes, de préférence entre environ 200 et environ 400 protéines d'intérêt différentes, par exemple environ 250 ou environ 300 protéines d'intérêt différentes, chaque protéine d'intérêt étant éventuellement associée à une ou à plusieurs sous-unités auxiliaires.

L'invention concerne un procédé de préparation d'une puce telle que décrite précédemment, ainsi que la puce susceptible d'être obtenue à l'aide d'un tel procédé, ledit procédé comprenant les étapes suivantes de clonage de l'ADNc ou des ADNc d'intérêt, d'expression de la ou des protéines (typiquement de la ou des protéines membranaires) codées par ledit ou lesdits ADNc dans des cellules en culture, de solubilisation des protéines exprimées à l'aide d'un détergent non dénaturant permettant la solubilisation desdites protéines tout en préservant leur conformation tridimensionnelle native et leur capacité de fixation à un support, et du dépôt desdites protéines solubilisées sur un support afin d'obtenir la puce d'intérêt. Contrairement à l'invention, la demande de brevet WO2004/108762 décrit des méthodes de préparation de membrane thyroïdienne solubilisée qui requièrent toutes une étape de fractionnement à l'aide de sels afin d'obtenir une fraction enrichie en protéines d'intérêt, ainsi qu'une étape d'ultracentrifugation.

L'invention concerne également l'utilisation d'une puce selon l'invention pour le criblage d'anticorps d'intérêt, de préférence d'un anticorps impliqué dans la survenue d'une maladie autoimmune, ou pour le diagnostic et/ou le suivi de l'évolution d'une maladie autoimmune.

Un autre objet de l'invention concerne par ailleurs un kit comprenant une puce telle que décrite précédemment, et, éventuellement, un ou plusieurs réactifs choisis de préférence parmi un tampon de blocage des sites non spécifiques, un tampon permettant l'association entre anticorps et antigènes (Ac/Ag), un tampon de lavage, un anticorps secondaire, un produit ou plusieurs produits de détection dudit ou desdits anticorps secondaires, et des instructions d'utilisation.

### DESCRIPTION DETAILLEE

### PRODUITS

L'invention concerne une puce à protéines permettant la détection d'anticorps, typiquement d'autoanticorps, de préférence d'autoanticorps dirigés contre des protéines membranaires et/ou complexes protéiques membranaires, dans un échantillon biologique, ainsi que son procédé d'obtention et ses utilisations. Plus précisément, l'invention concerne une puce à protéines permettant la détection d'autoanticorps, en particulier l'identification de nouveaux autoanticorps, dans un échantillon biologique. Les inventeurs décrivent, dans le cadre de la présente invention, une puce se présentant sous la forme d'un support comprenant au moins une protéine d'intérêt solubilisée (typiquement une protéine membranaire d'intérêt solubilisée), de préférence une protéine ayant conservé sa structure (ou conformation) tridimensionnelle native, et permettant la détection des complexes formés entre anticorps, typiquement autoanticorps, et épitopes spécifiques (épitopes séquentiels ou conformationnels), typiquement épitopes conformationnels, de ladite protéine d'intérêt.

La présente invention permet, pour la première fois, la détection, tout à la fois sensible et spécifique, des complexes formés entre autoanticorps et épitopes (séquentiels aussi bien que conformationnels) de la protéine membranaire d'intérêt (éventuellement associée à sa ou ses sous-unités auxiliaires), de préférence d'une protéine ou d'un complexe protéique impliqué ou suspecté d'être impliqué dans la survenue d'une encéphalopathie associée à des troubles de l'excitabilité neuronale. En outre, cette méthode rend facultative la dilution de l'échantillon biologique testé

Le terme « *échantillon biologique* » englobe tout échantillon (fluide, tissu ou cellule) dérivé d'un animal, typiquement d'un mammifère, d'un lagomorphe ou d'un rongeur (rat, souris, hamster, cochon d'Inde), de préférence d'un être humain. L'échantillon biologique est de préférence un fluide, typiquement un fluide contenant des autoanticorps produit par ledit animal. L'échantillon biologique peut être obtenu directement à partir dudit animal ou peut être dérivé d'une culture cellulaire obtenue à partir dudit animal. Les échantillons biologiques sont de préférence choisis par exemple parmi le sang, le sérum, le plasma, le liquide céphalo-rachidien (LCR), l'endolymphe de l'oreille interne, la périlymphe de l'oreille interne, et une sous-fraction ou un produit dérivés de ceux-ci. Un échantillon biologique préféré est un échantillon de liquide céphalo-rachidien. L'échantillon biologique peut avoir été soumis à un traitement tel qu'une dilution dans un véhicule acceptable. La présente invention permet toutefois avantageusement de se dispenser d'une telle étape.

Le terme « *autoanticorps* » comprend tout anticorps ainsi que tout fragment ou portions d'anticorps telles que Fab, F(ab')2, Fv et scFv se liant à un épitope d'une protéine du soi.

Le terme « *protéine* » couvre toute séquence d'acides aminés, ou ensemble de séquences d'acides aminés, comprenant au moins un antigène comprenant au moins un épitope. Dans le contexte de la présente invention, le terme « *protéine* » couvre typiquement une protéine membranaire ou un complexe protéique membranaire tel que défini précédemment.
Le terme « *protéine solubilisée* » désigne une protéine ayant été exposée à un détergent non dénaturant, i.e. un détergent non ionique (également identifié en tant que « détergent doux »), permettant la solubilisation desdites protéines tout en préservant leur conformation (structure tridimensionnelle) et leur capacité de fixation à un support. Cette solubilisation douce est la première étape utilisée pour la purification des protéines membranaires puis ultérieurement leur analyse fonctionnelle.
Le terme « *antigène* » désigne une macromolécule naturelle ou synthétique, reconnue par des anticorps ou des cellules du système immunitaire et capable d'engendrer une réponse immunitaire.
Le terme « *épitope* » (ou « *déterminant antigénique* ») désigne une séquence immunoréactive d'acides aminés, i.e., une séquence d'acides aminés capable de part sa structure d'interagir de manière spécifique avec un anticorps particulier.
Un même antigène peut comporter plusieurs épitopes (identiques ou différents) et ainsi induire des réponses immunitaires variées. Il existe des épitopes *séquentiels,* correspondant à une séquence d'acides aminés, et des épitopes *conformationnels,* liés à la structure authentique (conformation spatiale ou conformation tridimensionnelle native) de la protéine.
Au sens de l'invention, une protéine peut ainsi par exemple consister en un polypeptide simple chaine ; un ensemble de polypeptide reliés par des liaisons covalentes ou non ; une portion de protéine telle qu'une sous-unité, un domaine ou un fragment ; une protéine modifiée, par exemple par glycosylation (glycoprotéine) ou par combinaison à un lipide (lipoprotéine). Le terme « *protéine* » couvre également les variants, dérivés et analogues d'une protéine particulière.

Par « *variant* » ou « *dérivé* », on entend toute séquence d'acides aminés modifiée spontanément (variant naturel) ou volontairement (variant artificiel) par rapport à la séquence d'acides aminés spécifique codant la protéine particulière, de manière à ce que le variant protéique conserve au moins une des fonctions endogènes de ladite protéine particulière, par exemple l'ensemble des fonctions endogènes de cette protéine particulière (protéine équivalente sur le plan fonctionnel). Un variant peut être obtenu par addition ou insertion, délétion et/ou substitution d'un ou de plusieurs résidu(s) d'acide aminé au sein de la séquence d'acides aminés spécifique codant la protéine particulière.
Le choix de l'acide aminé de substitution peut se décider sur la base de similarité de polarité, charge, solubilité, hydrophobicité, hydrophilicité et/ou de nature amphiphatique garantissant la préservation d'une fonction particulière (e.g. fonction de transport ou de modulation de l'activité de transport). Les acides aminés chargés négativement incluent par exemple l'acide aspartique et l'acide glutamique, les acides aminés chargés positivement incluent par exemple la lysine et l'arginine et les acides aminés non chargés ayant un caractère hydrophile similaire incluent par exemple la leucine, l'isoleucine, la valine, la glycine, l'alanine, l'asparagine, la glutamine, la sérine, la thréonine, la phénylalanine et la tyrosine. Des substitutions conservatives peuvent également être faites, par exemple selon le Tableau 1 ci-dessous. Les acides aminés identifiés dans le même bloc dans la deuxième colonne et appartenant de préférence à la même ligne dans la troisième colonne peuvent par exemple se substituer les uns aux autres.

**Tableau 1 :**

| | | |
|---|---|---|
| ALIPHATIC | Non-polar | GAP |
| | | ILV |
| | Polar-Uncharged | CSTM |
| | | NQ |
| | Polar-charged | DE |
| | | KR |
| AROMATIC | | HFWY |

Par « *analogue* », on entend tout mimétique qui est un composé chimique existant dans la nature ou fabriqué artificiellement possédant au moins une des fonctions endogènes de la protéine qu'il imite, par exemple l'ensemble des fonctions endogènes de la protéine qu'il imite (protéine équivalente sur le plan fonctionnel).

Généralement, un épitope consiste en une séquence d'au moins 3 à 4 acides aminés, et plus couramment d'au moins 5 à 6 acides aminés. Les épitopes peuvent aussi consister en une séquence d'environ 7 à 8 acides aminés, ou même d'environ 10 acides aminés. Dans le cas d'un épitope séquentiel, les acides aminés sont consécutifs au sein de la séquence d'acides aminés linéaire de la protéine. Au contraire, comme expliqué précédemment, les acides aminés d'un épitope conformationnel ne sont pas nécessairement tous consécutifs au sein de la séquence d'acides aminés linéaire de la protéine, l'épitope conformationnel dépendant de la structure de la protéine. Dans le contexte de la présente invention, un épitope particulier d'une protéine donnée désigne une séquence d'acides aminés particulière (linéaire ou conformationnelle) ainsi que les équivalents immunologiques de cette dernière.

Dans un mode de réalisation particulier, l'invention concerne une puce à protéines permettant la détection d'anticorps, typiquement d'autoanticorps, dans un échantillon biologique, ladite puce comprenant au moins une protéine d'intérêt solubilisée (de préférence une protéine ayant conservé sa structure tridimensionnelle), typiquement au moins une protéine membranaire d'intérêt solubilisée choisie par exemple parmi une protéine canal ionique, une protéine transporteur, et une protéine récepteur membranaire régulant l'activité d'une protéine canal ionique ou d'une protéine transporteur, ladite protéine étant éventuellement, lorsqu'elle correspond à une sous-unité principale, associée à une ou plusieurs sous-unités auxiliaires, et ladite puce se présentant sous la forme d'un support permettant une détection des complexes formés entre anticorps et épitopes spécifiques de ladite protéine, cette dernière étant éventuellement associée à sa ou à ses sous-unités auxiliaires.
Le terme « *protéine canal ionique* » désigne une protéine membranaire qui permet le passage passif d'un ou plusieurs ions. Des protéines canal ionique susceptibles d'être fixées à la surface de la puce à protéines selon l'invention peuvent être choisies par exemple parmi les canaux ioniques et les récepteurs canaux.
Des exemples de canaux ioniques sont les canaux potassiques (canal potassique activé par le calcium intracellulaire, canal potassique à rectification entrante, canal potassique à deux domaines P, etc.), les canaux calciques, les canaux sodiques et les canaux chlores. Le canal ionique peut être un canal voltage-dépendant.

Le terme « *protéine transporteur* » désigne une protéine intrinsèque à la membrane cellulaire lipidique qui permet le passage sélectif de l'eau, d'ions et/ou de métabolites. Des protéines transporteur susceptibles d'être fixées à la surface de la puce à protéines selon l'invention peuvent être choisies par exemple parmi les aquaporines, les connexines, les Na+/K+-ATPases (ou pompes Na+/K+), les H+/K+-ATPases (ou pompes H+/K+), les échangeurs Na+/H+, les échangeurs Na+/Ca++, et les Na-K-Cl cotransporteurs.
Le terme « *protéine récepteur membranaire régulant l'activité d'une protéine canal ionique ou d'une protéine transporteur* » ou « *protéine récepteur membranaire régulatrice* » désigne toute protéine membranaire, associée ou non à une protéine canal ionique et/ou à une protéine transporteur, régulant au moins une activité desdites protéines. Des protéines régulatrices de type récepteur membranaire susceptibles d'être fixées à la surface de la puce à protéines selon l'invention peuvent être choisies par exemple parmi les récepteurs à sept domaines transmembranaires couplés aux protéines G et les récepteurs de type tyrosine kinase.

Comme indiqué ci-dessus, ladite au moins une protéine d'intérêt déposée sur le support correspond typiquement à une protéine simple brin (peptide) dans sa configuration tridimensionnelle, à la sous-unité principale d'une protéine multibrins (polypeptide) dans sa configuration naturelle, i.e. tridimensionnelle, ou encore à l'une des sous-unités principales d'une telle protéine multibrins. Cette protéine d'intérêt peut toutefois correspondre à ou être associée à une ou plusieurs sous-unités auxiliaires d'un ensemble de protéines associées sur le plan fonctionnel (complexe protéique).
Le terme « *sous-unité auxiliaire* » désigne les parties secondaires d'une protéine ou d'un complexe protéique, c'est-à-dire les parties autres que la ou les sous-unités principale(s). La sous-unité auxiliaire peut être membranaire ou périmembranaire. Souvent les sous-unités auxiliaires d'une protéine permettent de réguler l'activité (i.e. au moins l'une des fonctions) ou une propriété (i.e. une caractéristique fonctionnelle) de ladite protéine. Dans le cas d'un canal ionique ou d'une protéine transporteur, par exemple, la sous-unité principale permet généralement le passage d'ions ou de métabolites, tandis que les sous-unités auxiliaires régulent ce passage. Les protéines chaperonnes et les récepteurs ionotropiques (« ionophores ») sont des exemples de sous-unités auxiliaires d'un complexe protéique.

La protéine d'intérêt s'exprime dans les cellules du système nerveux d'un animal, typiquement d'un mammifère, d'un rongeur (rat, souris, hamster, cochon d'Inde) ou d'un lagomorphe, de préférence d'un être humain. La protéine d'intérêt est ainsi de préférence une protéine humaine.

De préférence, la protéine d'intérêt s'exprime dans les cellules du système nerveux d'un animal, typiquement d'un mammifère, d'un rongeur (rat, souris, hamster, cochon d'Inde) ou d'un lagomorphe, de préférence d'un être humain. La protéine d'intérêt est ainsi de préférence une protéine humaine. Il peut également s'agir d'une protéine provenant d'un animal distinct de l'homme tels ceux identifiés précédemment. De préférence, une telle protéine animale présente une séquence suffisamment proche de celle de l'isoforme humaine correspondante pour permettre la reconnaissance par les anticorps du système immunitaire d'un être humain d'au moins un épitope conformationnel de ladite protéine animale. Typiquement, la protéine animale d'intérêt présente une homologie entre sa séquence linéaire et celle de l'isoforme humaine d'au moins 50 %, de préférence d'au moins 75%, encore plus préférentiellement d'au moins 95%.

La protéine d'intérêt est typiquement une protéine membranaire humaine sélectionnée parmi un récepteur pentamérique (ou récepteur « cys-loop »), un récepteur de l'ATP, un récepteur ionotropique du glutamate, un canal voltage-dépendant, par exemple un canal calcique, sodique ou potassique voltage-dépendant, un canal potassique activé par le calcium intracellulaire, un canal potassique à rectification entrante, un canal potassique à deux domaines P, un canal ionique activable par les nucléotides cycliques, un canal ionique de type TRP (Transient Receptor Potential), un canal chlore, un canal cationique sensible à l'acidité, une sous-unité auxiliaire de canal sodique, une sous-unité auxiliaire de canal calcique.
Comme indiqué précédemment, la protéine d'intérêt peut également être une protéine de rongeur, typiquement de souris ou de rat. Des protéines membranaires d'intérêt provenant de rongeurs peuvent être par exemple choisies parmi un canal sodique de rat, un canal calcique de rat et un canal glutamate de rat.

Dans le cadre de la présente invention, la protéine d'intérêt, typiquement la protéine membranaire d'intérêt, est de préférence associée (ou suspectée d'être associée) à une pathologie humaine responsable de l'apparition d'autoanticorps.

Dans un mode de réalisation particulier de l'invention, la protéine d'intérêt, typiquement la protéine membranaire d'intérêt, est avantageusement associée (ou suspectée d'être associée) à une maladie ou pathologie auto-immune (ou présumée auto-immune) affectant le système nerveux (maladie neurologique).

Des exemples de pathologies auto-immunes affectant le système nerveux comprennent les encéphalopathies associées à des troubles de l'excitabilité neuronale, typiquement à des troubles de l'excitabilité de récepteurs neuronaux, et incluent, sans y être limités, les syndromes épileptiques acquis, les encéphalites, les syndromes neurologiques paranéoplasiques (SNP), et les syndromes neurologiques non paranéoplasiques avec autoanticorps. Des exemples d'encéphalopathies auto-immunes (ou présumées auto-immunes), convulsivantes ou non, associées à des troubles de l'excitabilité neuronale sont l'encéphalite limbique (qui implique des autoanticorps dirigés contre des protéines associées aux canaux potassiques voltage-dépendants ou des autoanticorps directement dirigés contre ces canaux), l'encéphalite de Rasmussen (associée à des autoanticorps dirigés contre des récepteurs au glutamate et à l'acétylcholine), les encéphalites post-infectieuses, l'encéphalite à anticorps anti-récepteurs NMDA (anti-NMDAR), le syndrome de Morvan, la maladie de Devic ou neuromyélite optique de Devic (qui implique des autoanticorps dirigés contre l'aquaporine 4), les syndromes opso-myocloniques de l'enfant (d'origine post-infectieuse ou paranéoplasique), les encéphalomyélites, les syndromes épileptiques acquis, et les syndromes neurologiques paranéoplasiques (SNP). Préférentiellement, l'encéphalopathie est une atteinte du système nerveux central pouvant être associée à des troubles de l'excitabilité neuronale d'origine autoimmune (démontrée ou suspectée) et d'origine post-infectieuse ou paranéoplasique.

Dans un autre mode de réalisation particulier de l'invention, la protéine d'intérêt, typiquement la protéine membranaire d'intérêt, est une protéine associée (ou suspectée d'être associée) à un syndrome neurologique périphérique (éventuellement associé à une encéphalopathie) d'origine autoimmune (démontrée ou suspectée), et, typiquement, d'origine post-infectieuse ou paranéoplasique. Les syndromes neurologiques périphériques peuvent être à l'origine de paralysies musculaires d'origine pré- ou post-synaptique. Le syndrome d'Isaac est un exemple de syndrome neurologique périphérique.

Dans un autre mode de réalisation particulier de l'invention, la protéine d'intérêt, typiquement la protéine membranaire d'intérêt, est une protéine associée (ou suspectée d'être associée) à une maladie neurosensorielle (associée ou non à des troubles de l'excitabilité de récepteurs sensoriels), par exemple à une maladie de l'oreille interne telles que la maladie de Menière ou des surdités acquises ou présumées congénitales.

Un puce particulière selon l'invention comprend au moins une protéine membranaire d'intérêt, typiquement plusieurs protéines membranaires d'intérêt, ladite protéine étant codée par une séquence d'ADNc humain choisie parmi les séquences nucléotidiques identifiées dans le tableau 2 ci-dessous ou correspondant à une séquence d'acides aminés choisie parmi les séquences d'acides aminés également identifiées dans le tableau 2 ci-dessous, de préférence parmi les séquences d'acides aminés SEQ ID NO :1 à SEQ ID NO :311.

**Tableau 2 :**

| **Gène** | **Nom complet du gène** | **Ref Seq Nucleotide (isoformes)** | **Taille du gène amplifié (bp)** | **Ref Seq Protein (isoformes) et SEQ ID NO associés** |
|---|---|---|---|---|
| **Récepteurs "Cys-loop"** | | | | |
| HTR3A | serotonin 5-HT3 receptor A subunit | NM_000869 (Hs), NM_213621 (Hs), NM_013561 (Mm), NM_001099644 (Mm), NM_024394 (Rn) | 1537 | NP_000860 (Hs) (SEQ ID NO: 1), NP_998786 (Hs) (SEQ ID NO: 2), NP_038589 (Mm), NP_001093114 (Mm), NP 077370 (Rn) |
| HTR3B | serotonin 5-HT3 receptor B subunit | NM_006028 (Hs), NM_020274 (Mm), NM_022189 (Rn) | 1333 | NP_006019 (Hs) (SEQ ID NO: 3), NP_064670 (Mm), NP_071525 (Rn) |
| HTR3C | serotonin 5-HT3 receptor C subunit | NM_130770 | 1348 | NP_570126 (Hs) (SEQ ID NO: 4) |
| HTR3D | serotonin 5-HT3 receptor D subunit | NM_182537 | 844 | NP_872343 (Hs) (SEQ ID NO: 5) |
| HTR3E | serotonin 5-HT3 receptor E subunit | NM_182589 | 1420 | NP_872395 (Hs) (SEQ ID NO: 6) |
| CHRNA1 | nicotinic acetylcholine receptor α1 subunit | NM_001039523 (Hs), NM_007389 (Mm), NM_024485 (Rn) | 1450 | NP_001034612 (Hs) (SEQ ID NO: 7), NP_031415 (Mm), NP_077811 (Rn) |
| CHRNA2 | nicotinic acetylcholine receptor α2 subunit | NM_000742 (Hs), NM_144803 (Mm), NM_133420 (Rn) | 1591 | NP_000733 (Hs) (SEQ ID NO: 8), NP_659052 (Mm), NP_596911 (Rn) |
| CHRNA3 | nicotinic acetylcholine receptor α3 subunit | NM_000743 (Hs), NM_145129 (Mm), NM_052805 (Rn) | 1519 | NP_000734 (Hs) (SEQ ID NO: 9), NP_660111 (Mm), NP_434692 (Rn) |
| CHRNA4 | nicotinic acetylcholine receptor α4 subunit | NM_000744 (Hs), NM_015730 (Mm), NM_024354 (Rn) | 1885 | NP_000735 (Hs) (SEQ ID NO: 10), NP_056545 (Mm), NP_077330 (Rn) |
| CHRNA5 | nicotinic acetylcholine receptor α5 subunit | NM_000745 (Hs), NM _176844 (Mm), NM_017078 (Rn) | 1407 | NP_000736 (Hs) (SEQ ID NO: 11), NP_789814 (Mm), NP_058774 (Rn) |
| CHRNA6 | nicotinic acetylcholine receptor α6 subunit | NM_004198 (Hs), NM_021369 (Mm), NM_057184 (Rn) | 1489 | NP_004189 (Hs) (SEQ ID NO: 12), NP_067344 (Mm), NP_476532 (Rn) |
| CHRNA7 | nicotinic acetylcholine receptor α7 subunit | NM_000746 (Hs), NM_007390 (Mm), NM_012832 (Rn) | 1513 | NP_000737 (Hs) (SEQ ID NO: 13), NP_031416 (Mm), NP_036964 (Rn) |
| CHRNA9 | nicotinic acetylcholine receptor α9 subunit | NM 017581 (Hs), NM_001081104 (Mm), NM_022930 (Rn) | 1444 | NP_060051 (Hs) (SEQ ID NO: 14), NP_001074573 (Mm), NP_075219 (Rn) |
| CHRNA10 | nicotinic acetylcholine receptor α10 subunit | NM_020402 (Hs), NM_001081424 (Mm), NM_ 022639 (Rn) | 1354 | NP_065135 (Hs) (SEQ ID NO: 15), NP_001074893 (Mm), NP_072161 (Rn) |
| CHRNB1 | nicotinic acetylcholine receptor β1 subunit | NM_000747 (Hs), NM_009601 (Mm), NM_012528 (Rn) | 1510 | NP_000738 (Hs) (SEQ ID NO: 16), NP_033731 (Mm), NP_036660 (Rn) |
| CHRNB2 | nicotinic acetylcholine receptor β2 subunit | NM_000748 (Hs), NM_009602 (Mm), NM_019297 (Rn) | 1510 | NP_000739 (Hs) (SEQ ID NO: 17), NP_033732 (Mm), NP_062170 (Rn) |
| CHRNB3 | nicotinic acetylcholine receptor β3 subunit | NM_000749 (Hs), NM_173212 (Mm), NM_133597 (Rn) | 1381 | NP_000740 (Hs) (SEQ ID NO: 18), NP_775304 (Mm), NP_598281 (Rn) |
| CHRNB4 | nicotinic acetylcholine receptor β4 subunit | NM_000750 (Hs), NM_148944 (Mm), NM_052806 (Rn) | 1501 | NP_000741 (Hs) (SEQ ID NO: 19), NP_683746 (Mm), NP_434693 (Rn) |
| CHRND | nicotinic acetylcholine receptor δ subunit | NM_000751 (Hs), NM_021600 (Mm), NM_019298 (Rn) | 1555 | NP_000742 (Hs) (SEQ ID NO: 20), NP_067611 (Mm), NP_062171 (Rn) |
| CHRNE | nicotinic acetylcholine receptor ε subunit | NM_000080 (Hs), NM_009603 (Mm), NM_017194 (Rn) | 1483 | NP_000071 (Hs) (SEQ ID NO: 21), NP_033733 (Mm), NP_058890 (Rn) |
| CHRNG | nicotinic acetylcholine receptor γ subunit | NM_005199 (Hs), NM_009604 (Mm), NM_019145 (Rn) | 1558 | NP_005190 (Hs) (SEQ ID NO: 22), NP _033734 (Mm), NP _062018 (Rn) |
| GABRA1 | GABA receptor α1 subunit | NM_000806 (Hs), NM_010250 (Mm), NM_183326 (Rn) | 1375 | NP_000797 (Hs) (SEQ ID NO: 23), NP_034380 (Mm), NP_899155 (Rn) |
| GABRA2 | GABA receptor α2 subunit | NM_000807 (Hs), NM_008066 | 1360 | NP_000798 (Hs) (SEQ ID NO: 24), NP_032092 (Mm) |
| GABRA3 | GABA receptor α3 subunit | NM 000808 (Hs), NM_008067 (Mm), NM_017069 (Rn) | 1483 | NP_000799 (Hs) (SEQ ID NO: 25), NP_032093 (Mm), NP_058765 (Rn) |
| GABRA4 | GABA receptor α4 subunit | NM 000809 (Hs), NM_010251 (Mm), NM_080587 (Rn) | 1666 | NP_000800 (Hs) (SEQ ID NO: 26), NP_034381 (Mm), NP_542154 (Rn) |
| GABRA5 | GABA receptor α5 subunit | NM 000810 (Hs), NM_176942 (Mm), NM_017295 (Rn) | 1390 | NP_000801 (Hs) (SEQ ID NO: 27), NP_795916 (Mm), NP_058991 (Rn) |
| GABRA6 | GABA receptor α6 subunit | NM_000811 (Hs), NM_001099641 (Mm), NM_021841 (Rn) | 1363 | NP_000802 (Hs) (SEQ ID NO: 28), NP_001093111 (Mm), NP_068613 (Rn) |
| GABRB1 | GABA receptor β1 subunit | NM 000812 (Hs), NM_008069 (Mm), NM_012956 (Rn) | 1429 | NP_000803 (Hs) (SEQ ID NO: 29), NP_032095 (Mm), NP_037088 (Rn) |
| GABRB2 | GABA receptor β2 subunit | NM_021911 (Hs), NM_000813 (Hs), NM_008070 (Mm), NM_012957 (Rn) | 1543 | NP_068711 (Hs) (SEQ ID NO: 30), NP_000804 (Hs) (SEQ ID NO: 31), NP_032096 (Mm), NP_037089 (Rn) |
| GABRB3 | GABA receptor β3 subunit | NM 000814 (Hs), NM_008071 (Mm), NM_017065 (Rn) | 1426 | NP_000805 (Hs) (SEQ ID NO: 32), NP_032097 (Mm), NP_058761 (Rn) |
| GABRD | GABA receptor δ subunit | NM_000815 (Hs), NM_008072 (Mm), NM_017289 (Rn) | 1360 | NP_000806 (Hs) (SEQ ID NO: 33), NP_032098 (Mm), NP_058985 (Rn) |
| GABRE* | GABA receptor ε subunit * | NM_004961 (Hs), NM_017369 (Mm), NM_023091 (Rn) | 1525 | NP_004952 (Hs) (SEQ ID NO: 34), NP _059065 (Mm), NP _075579 (Rn) |
| GABRG1 | GABA receptor γ1 subunit | NM_173536 (Hs), NM_010252 (Mm), NM_080586 (Rn) | 1399 | NP_775807 (Hs) (SEQ ID NO: 35), NP_034382 (Mm), NP_542153 (Rn) |
| GABRG2 | GABA receptor γ2 subunit | NM_198904 (Hs), NM_008073 (Mm), NM_183327 (Rn) | 1432 | NP_944494 (Hs) (SEQ ID NO: 36), NP_032099 (Mm), NP_899156 (Rn) |
| GABRG3 | GABA receptor γ 3 subunit | NM 033223 (Hs), NM_008074 (Mm), NM_024370 (Rn) | 1405 | NP_150092 (Hs) (SEQ ID NO: 37), NP_032100 (Mm), NP_077346 (Rn) |
| GABRP | GABA receptor π subunit | NM_014211 (Hs), NM_146017 (Mm), NM_031029 (Rn) | 1183 | NP_055026 (Hs) (SEQ ID NO: 38), NP_666129 (Mm), NP_112291 (Rn) |
| GABRR1 | GABA receptor ρ1 subunit | NM 002042 (Hs), NM_008075 (Mm), NM_017291 (Rn) | 1322 | NP_002033 (Hs) (SEQ ID NO: 39), NP_032101 (Mm), NP_058987 (Rn) |
| GABRR2 | GABA receptor ρ2 subunit | NM 002043 (Hs), NM_008076 (Mm), NM_017292 (Rn) | 1474 | NP _002034 (Hs) (SEQ ID NO: 40), NP_032102 (Mm), NP_058988 (Rn) |
| GABRR3 | GABA receptor ρ3 subunit | NM_001105580 (Hs), NM_001081190 (Mm), NM_138897 (Rn) | 1405 | NP_001099050 (Hs) (SEQ ID NO: 41), NP_001074659 (Mm), NP_620252 (Rn) |
| GABRQ | GABA receptor θ subunit | NM 018558 (Hs), NM_020488 (Mm), NM_031733 (Rn) | 1183 | NP_061028 (Hs) (SEQ ID NO: 42), NP_065234 (Mm), NP_113921 (Rn) |
| GLRA1 | glycine receptor α1 subunit | NM 000171 (Hs), NM_020492 (Mm), NM_013133 (Rn) | 1354 | NP _000162 (Hs) (SEQ ID NO: 43), NP_065238 (Mm), NP_037265 (Rn) |
| GLRA2 | glycine receptor α2 subunit | NM 002063 (Hs), NM_183427 (Mm), NM_012568 (Rn) | 1363 | NP_002054 (Hs) (SEQ ID NO: 44), NP_906272 (Mm), NP_036700 (Rn) |
| GLRA3 | glycine receptor α3 subunit | NM 006529 (Hs), NM_080438 (Mm), NM_053724 (Rn) | 1396 | NP_006520 (Hs) (SEQ ID NO: 45), NP_536686 (Mm), NP_446176 (Rn) |
| GLRA4 | glycine receptor α4 subunit | NM_001024452.2 (Hs), NM_010297 (Mm) | 1135 | NP_001019623 (Hs) (SEQ ID NO: 46), NP_034427 (Mm), XP_346351.2 (Rn) |
| GLRB | glycine receptor β subunit | NM 000824 (Hs), NM_010298 (Mm), NM_053296 (Rn) | 1498 | NP_000815 (Hs) (SEQ ID NO: 47), NP_034428 (Mm), NP_445748 (Rn) |

| **Récepteurs de l'ATP** | | | | |
|---|---|---|---|---|
| P2RX1 | ATP receptor P2X1 subunit | NM_002558 (Hs), NM_008771 (Mm), NM_012997 (Rn), AF231010 (Rn) | 1201 | NP_002549 (Hs) (SEQ ID NO: 48), NP_032797 (Mm), NP_037129 (Rn), Q9JIF8 (Rn) |
| P2RX2 | ATP receptor P2X2 subunit Isoform A | AF190824 (Hs), AF190823 (Hs), AF190825 (Hs), NM_170682 (Hs), NM_153400 (Mm), NM_053656 (Rn) | 1417 | AAF19173 (Hs) (SEQ ID NO: 49), NP_733782 (Hs) (SEQ ID NO: 50), AAF19171 (Hs) (SEQ ID NO: 51), AAF19172 (Hs) (SEQ ID NO: 52), NP_700449 (Mm), NP_446108 (Rn) |
| P2RX3 | ATP receptor P2X3 subunit | NM_002559 (Hs), NM_145526 (Mm), NM_031075 (Rn) | 1198 | NP_002550 (Hs) (SEQ ID NO: 53), NP_663501 (Mm), NP_112337 (Rn) |
| P2RX4 | ATP receptor P2X4 subunit | NM_002560 (Hs), NM_011026 (Mm), NM_031594 (Rn) | 1168 | NP_002551 (Hs) (SEQ ID NO: 54), NP_035156 (Mm), NP_113782 (Rn) |
| P2RX5 | ATP receptor P2X5 subunit isoform A | NM_002561 (Hs), NM_033321 (Mm), NM_080780 (Rn) | 1270 | NP_002552 (Hs) (SEQ ID NO: 55), NP_201578 (Mm), NP_542958 (Rn) |
| P2RXL1 | ATP receptor P2X6 subunit (aka P2XM, and P2X-like 1) | NM_005446 (Hs), NM_011028 (Mm), NM_012721 (Rn) | 1330 | NP_005437 (Hs) (SEQ ID NO: 56), NP_035158 (Mm), NP_036853 (Rn) |
| P2RX7 | ATP receptor P2X7 subunit | NM_002562 (Hs), NM_011027 (Mm), FJ436444 (Mm), NM_019256 (Rn), FJ436445 (Rn) | 1792 | NP_002553 (Hs) (SEQ ID NO: 57), NP_035157 (Mm), ACR61395 (Mm), ACR61396 (Rn), NP_062129 (Rn) |

| **Récepteurs ionotropiques du glutamate** | | | | |
|---|---|---|---|---|
| Orphan GRID1 | glutamate receptor δ1 subunit | NM_017551 (Hs), NM_008166 (Mm), NM_024378 (Rn) | 3031 | NP_060021 (Hs) (SEQ ID NO: 58), NP_032192 (Mm), NP_077354 (Rn) |
| Orphan GRID2 | glutamate receptor δ2 subunit | NM_001510 (Hs), NM_008167 (Mm), NM_024379 (Rn) | 3025 | NP_001501 (Hs) (SEQ ID NO: 59), NP_032193 (Mm), NP_077355 (Rn) |
| Kainate GRIK4 | glutamate receptor KA1 subunit (aka EAA1) | NM_014619 (Hs), NM_175481 (Mm), NM_012572 (Rn) | 1875 | NP_055434 (Hs) (SEQ ID NO: 60), NP_780690 (Mm), NP_036704 (Rn) |
| Kainate GRIK5 | glutamate receptor KA2 subunit (aka EAA2) | NM_002088 (Hs), NM_008168 (Mm), NM_031508 (Rn) | 2947 | NP_002079 (Hs) (SEQ ID NO: 61), NP_032194 (Mm), NP_113696 (Rn) |
| NMDA GRIN1 | glutamate receptor NMDAR1 subunit | NM_007327 (Hs), NM_008169 (Mm), NM_017010 (Rn) | 2665 | NP_015566 (Hs) (SEQ ID NO: 62), NP_032195 (Mm), NP_058706 (Rn) |
| NMDA GRIN2A | glutamate receptor NMDAR2A subunit | NM_000833 (Hs), NM_008170 (Mm), NM_012573 (Rn) | 4396 | NP_000824 (Hs) (SEQ ID NO: 63), NP_032196 (Mm), NP_036705 (Rn) |
| NMDA GRIN2B | glutamate receptor NMDAR2B subunit | NM_000834 (Hs), NM_008171 (Mm), NM_012574 (Rn) | 4459 | NP_000825 (Hs) (SEQ ID NO: 64), NP_032197 (Mm), NP_036706 (Rn) |
| NMDA GRIN2C | glutamate receptor NMDAR2C subunit | NM_000835 (Hs), NM 010350 (Mm), NM_012575 (Rn) | 3706 | NP_000826 (Hs) (SEQ ID NO: 65), NP_034480 (Mm), NP_036707 (Rn) |
| NMDA GRIN2D | glutamate receptor NMDAR2D subunit | NM_000836 (Hs), NM_008172 (Mm), NM_022797 (Rn) | 4015 | NP_000827 (Hs) (SEQ ID NO: 66), NP_032198 (Mm), NP_073634 (Rn) |
| NMDA GRIN3A | glutamate receptor NMDAR3A subunit | NM_133445 (Hs), NM_001033351 (Mm) | 3352 | NP_597702 (Hs) (SEQ ID NO: 67), NP_001028523 (Mm) |
| NMDA GRIN3B | glutamate receptor NMDAR3B subunit (aka χ-2) | NM_138690 (Hs), NM_130455 (Mm), NM_133308 (Rn) | 3133 | NP_619635 (Hs) (SEQ ID NO: 68), NP_569722 (Mm), NP_579842 (Rn) |
| AMPA GRIA1 | glutamate receptor GLUR1 subunit (aka KR4, HBGR1, GluHI) | NM_000827 (Hs), NM_001114183 (Hs), NM 008165 (Mm), NM_001113325 (Mm), NM_031608 (Rn) | 2725 | NP_000818 (Hs) (SEQ ID NO: 69), NP_001107655 (Hs) (SEQ ID NO: 70), NP 001106796 (Mm), NP_032191 (Mm), NP_113796 (Rn) |
| AMPA GRIA2 | glutamate receptor GLUR2 subunit | NM_001083619 (Hs), NM_000826 (Hs), NM 013540 (Mm), NM_001083806 (Mm), NM_001039195 (Mm), NM_017261 (Rn), NM_001083811 (Rn) | 2656 | NP_000817 (Hs) (SEQ ID NO: 71), NP_001077088 (Hs) (SEQ ID NO: 72), NP_001077275 (Mm), NP_001034284 (Mm), NP 038568 (Mm), NP_058957 (Rn), NP_001077280 (Rn) |
| AMPA GRIA3 | glutamate receptor GLUR3 subunit (aka GLURC) | NM_007325 (Hs), NM_000828 (Hs), NM_016886 (Mm), NM_001112742 (Rn), NM_ 032990 (Rn) | 2686 | NP_015564 (Hs) (SEQ ID NO: 73), NP_000819 (Hs) (SEQ ID NO: 74), NP_058582 (Mm), NP_116785 (Rn), NP_001106213 (Rn) |
| AMPA GRIA4 | glutamate receptor GluR4 subunit | NM_001077243 (Hs), NM_000829 (Hs), NM_001077243 (Hs), NM_000829 (Hs), NM_001113180 (Mm), NM_019691 (Mm), NM_001113184 (Rn), NM_ 017263 (Rn) | 2713 | NP_000820 (Hs) (SEQ ID NO: 75), NP_001070711 (Hs) (SEQ ID NO: 76), NP_000820 (Hs), NP_001070711 (Hs), NP_062665 (Mm), NP _001106651 (Mm), NP_058959 (Rn), NP_001106655 (Rn) |
| Kainate GRIK1 | glutamate receptor GLUR5 subunit (aka EEA3) | NM_175611 (Hs), NM_010348 (Mm), NM_017241 (Rn) | 2761 | NP_783300 (Hs) (SEQ ID NO: 77), NP_034478 (Mm), NP_058937 (Rn) |
| Kainate GRIK2 | glutamate receptor GLUR6 subunit (aka EAA4 aka β2) | NM_021956 (Hs), NM_010349 (Mm), NM_019309 (Rn) | 2731 | NP_068775 (Hs) (SEQ ID NO: 78), NP_034479 (Mm), NP_062182 (Rn) |
| Kainate GRIK3 | glutamate receptor GLUR7 subunit (aka EAA5) | NM_000831 (Hs), NM_001081097 (Mm), NM_001112716 (Rn) | 2764 | NP_000822 (Hs) (SEQ ID NO: 79), NP_001074566 (Mm), NP_00 1106187 (Rn) |

| **Canaux sodiques dépendants du voltage** | | | | |
|---|---|---|---|---|
| SCN1A | voltage gated sodium channel type I a subunit | NM_006920 (Hs), NM_018733 (Mm), NM_030875 (Rn) | 5998 | NP_008851 (Hs) (SEQ ID NO: 80), NP_061203 (Mm) (SEQ ID NO: 81), NP_110502 (Rn) (SEQ ID NO: 82) |
| | | | | |
| SCN2A | voltage gated sodium channel type II a subunit | NM_001040143 (Hs), NM_001040142 (Hs), NM_001099298 (Mm), NM_ 012647 (Rn) | 6019 | NP_001035232 (Hs) (SEQ ID NO: 83), NP_001035233 (Hs) (SEQ ID NO: 84), NP_001092768 (Mm) (SEQ ID NO: 85), NP 036779 (Rn) (SEQ ID NO: 86) |
| SCN3A | voltage gated sodium channel type III a subunit | NM_006922 (Hs) NM_018732 (Mm), NM_013119(Rn) | 6004 | NP_008853 (Hs) (SEQ ID NO: 87), NP_061202 (Mm) (SEQ ID NO :88), NP_037251 (Rn) (SEQ ID NO: 89) |
| SCN4A | voltage gated sodium channel type IV a subunit | NM_000334 (Hs), NM_133199 (Mm), NM_013178 (Rn) | 5512 | NP_000325 (Hs) (SEQ ID NO: 90), NP_573462 (Mm) (SEQ ID NO: 91), NP _ 037310 (Rn) (SEQ ID NO: 92) |
| SCN5A | voltage gated sodium channel type V a subunit | NM_001099404 (Hs), NM_000335 (Hs), NM_198056 (Hs), NM_001099405 (Hs), NM_021544 (Mm), NM_013125 (Rn) | 6055 | NP_001092874 (Hs) (SEQ ID NO: 93), NP_001092875 (Hs) (SEQ ID NO: 94), NP000326 (Hs) (SEQ ID NO: 95), NP_932173 (Hs) (SEQ ID NO: 96), NP_067519 (Mm) (SEQ ID NO: 97), NP_ 037257 (Rn) (SEQ ID NO: 98) |
| SCN8A | voltage gated sodium channel type VIII a subunit | NM_014191 (Hs), NM_001077499 (Mm), NM_019266 (Rn) | 5944 | NP_055006 (Hs) (SEQ ID NO: 99), NP_001070967 (Mm) (SEQ ID NO: 100), NP_062139 (Rn) (SEQ ID NO: 101) |
| SCN9A | voltage gated sodium channel type IX a subunit | NM_002977 (Hs) NM 018852 (Mm), NM_133289 (Rn) | 5935 | NP_002968 (Hs) (SEQ ID NO: 102), NP_061340 (Mm) (SEQ ID NO: 103), NP_579823 (Rn) (SEQ ID NO: 104) |
| SCN10A | voltage gated sodium channel type X a subunit | NM_006514 (Hs), NM_009134 (Mm), NM_017247 (Rn) | 5872 | NP_006505 (Hs) (SEQ ID NO: 105), NP_033160 (Mm) (SEQ ID NO: 106), NP_058943 (Rn) (SEQ ID NO: 107) |
| SCN11A | voltage gated sodium channel type XI a subunit | NM_014139 (Hs), NM_011887 (Mm), NM_019265 (Rn) | 5377 | NP_054858 (Hs) (SEQ ID NO: 108), NP_036017 (Mm) (SEQ ID NO: 109), NP_062138 (Rn) (SEQ ID NO: 110) |

| **Canaux calciques dépendants du voltage** | | | | |
|---|---|---|---|---|
| CACNA1S | voltage gated calcium channel L-type a1S subunit | NM_000069 (Hs), NM_001081023 (Mm), NM_053873 (Rn) | 5626 | NP_000060 (Hs) (SEQ ID NO: 111), NP_001074492 (Mm) (SEQ ID NO: 112), NP_446325 (Rn) (SEQ ID NO: 113) |
| CACNA1C | voltage gated calcium channel L-type a1C subunit | NM_000719 (Hs), NM_009781 (Mm), NM_012517 (Rn) | 6418 | NP_000710 (Hs) (SEQ ID NO: 114), NP_033911 (Mm) (SEQ ID NO: 115), NP_036649 (Rn) (SEQ ID NO: 116) |
| CACNA1D | voltage gated calcium channel L-type a1D subunit | NM_000720 (Hs), NM_028981 (Mm), NM_(Rn) | 017298 6550 | NP_000711 (Hs) (SEQ ID NO: 117), NP_083257 (Mm) (SEQ ID NO: 118), NP_058994 (Rn) (SEQ ID NO: 119) |
| CACNA1F | voltage gated calcium channel L-type a1F subunit | NM_005183 (Hs), NM 019582 (Mm), NM_053701 (Rn) | 5938 | NP_005174 (Hs) (SEQ ID NO: 120), NP_062528 (Mm) (SEQ ID NO: 121), NP_446153 (Rn) (SEQ ID NO: 122) |
| CACNA1A | voltage gated calcium channel P/Q-type a1A subunit | NM_023035 (Hs), NM_000068.3 (Hs), NM_007578 (Mm), NM_012918 (Rn) | 6802 | NP_075461 (Hs) (SEQ ID NO: 123), NP_000059 (Hs) (SEQ ID NO: 124), NP_031604 (Mm) (SEQ ID NO: 125), NP 037050 (Rn) (SEQ ID NO: 126) |
| CACNA1B | voltage gated calcium channel N-type a1B subunit | NM_000718 (Hs), NM_001042528 (Mm), NM_147141 (Rn) | 7021 | NP_000709 (Hs) (SEQ ID NO: 127), NP_001035993 (Mm) (SEQ ID NO: 128), NP_671482 (Rn) (SEQ ID NO: 129) |
| CACNA1E | voltage gated calcium channel R-type a1E subunit | NM 000721 (Hs), NM_009782 (Mm), NM_019294 (Rn) | 6814 | NP_000712 (Hs) (SEQ ID NO: 130), NP_033912 (Mm) (SEQ ID NO: 131), NP_062167 (Rn) (SEQ ID NO: 132) |
| CACNA1G | voltage gated calcium channel T-type a1G subunit | NM_198378 (Hs), NM_198387 (Hs), NM_198379 (Hs), NM_938190 (Hs), NM_009783 (Mm), NM_031601 (Rn) | 7135 | NP_061496 (Hs) (SEQ ID NO: 133), NP_938192 (Hs) (SEQ ID NO: 134), NP_938190 (Hs) (SEQ ID NO: 135), NP_938201 (Hs) (SEQ ID NO: 136), NP_938193 (Hs) (SEQ ID NO: 137), NP_033913 (Mm) (SEQ ID NO: 138), NP_ 113789 (Rn) (SEQ ID NO: 139) |
| CACNA1H | voltage gated calcium channel T-type a1H subunit | NM_021098 (Hs), NM_021415 (Mm), NM_153814 (Rn) | 7069 | NP_066921 (Hs) (SEQ ID NO: 140), NP_067390 (Mm) (SEQ ID NO: 141), NP_722521 (Rn) (SEQ ID NO: 142) |
| CACNA1I | voltage gated calcium channel T-type a1I subunit | NM_001003406 (Hs), NM_021096 (Hs), NM_001044308 (Mm), (Rn) | NM_020084 6673 | NP_066919 (Hs) (SEQ ID NO: 143), NP_001003406 (Hs) (SEQ ID NO: 144), NP_001037773 (Mm) (SEQ ID NO: 145), NP_064469 (Rn) (SEQ ID NO: 146) |

| **Canaux potassiques dépendants du voltage** | | | | |
|---|---|---|---|---|
| Shaker KCNA1 | voltage gated Potassium channel Delayed Rectifier member 1 | NM_000217 (Hs), NM_010595 (Mm), NM_173095 (Rn) | 1492 | NP 000208 (Hs) (SEQ ID NO: 147), NP_034725 (Mm), NP_775118 (Rn) |
| Shaker KCNA2 | voltage gated Potassium channel Delayed Rectifier member 2 | NM _004974 (Hs), NM_008417 (Mm), NM_012970 (Rn) | 1504 | NP_004965 (Hs) (SEQ ID NO: 148), NP_032443 (Mm), NP_037102 (Rn) |
| Shaker KCNA3 | voltage gated Potassium channel Delayed Rectifier member 3 | NM_002232 (Hs), NM_008418 (Mm), NM_019270 (Rn) | 1729 | NP_ 002223 (Hs) (SEQ ID NO: 149), NP_032444 (Mm), NP_062143 (Rn) |
| Shaker KCNA4 | voltage gated Potassium channel A-Type, Fast inactivation member 4 | NM_002233 (Hs), NM_021275 (Mm), NM_012971 (Rn) | 1963 | NP_002224 (Hs) (SEQ ID NO: 150), NP_067250 (Mm), NP_037103 (Rn) |
| Shaker KCNA5 | voltage gated Potassium channel Delayed Rectifier member 5 | NM_002234 (Hs), NM_145983 (Mm), NM_012972 (Rn) | 1843 | NP_002225 (Hs) (SEQ ID NO: 151), NP_666095 (Mm), NP_037104 (Rn) |
| Shaker KCNA6 | voltage gated Potassium channel Delayed Rectifier member 6 | NM_002235 (Hs), NM_013568 (Mm), NM_023954 (Rn) | 1597 | NP_002226 (Hs) (SEQ ID NO: 152), NP_038596 (Mm), NP_076444 (Rn) |
| Shaker KCNA7 | voltage gated Potassium channel Delayed Rectifier member 7 | NM_031886 (Hs), NM_010596 (Mm), NM_001108914 (Rn) | 1375 | NP_114092 (Hs) (SEQ ID NO: 153), NP_034726 (Mm), NP_001102384 |
| Shaker KCNA10 | voltage gated Potassium channel Delayed Rectifier member 10 | NM_005549 (Hs), NM_001074609 (Mm), XM_227577 (Rn) | 1537 | NP_005540 (Hs) (SEQ ID NO: 154), NP_001074609 (Mm), XP_227577 (Rn) |
| Shab KCNB1 | voltage gated Potassium channel Delayed Rectifier Shab Related member 1 | NM_004975 (Hs), NM_008420 (Mm), NM_013186 (Rn) | 2581 | NP_004966 (Hs) (SEQ ID NO: 155), NP_032446 (Mm), NP_037318 (Rn) |
| Shab KCNB2 | voltage gated Potassium channel Delayed Rectifier Shab Related member 2 | NM_004770 (Hs), NM_001098528 (Mm), NM_054000 (Rn) | 2737 | NP_004761 (Hs) (SEQ ID NO: 156), NP_001091998 (Mm), NP _446452 (Rn) |
| Shaw KCNC1 | voltage gated Potassium channel Delayed Rectifier Shaw Related member 1 | NM_004976 (Hs), NM_008421 (Mm), NM_001112739 (Mm), NM_139217.1 (Rn), NM_012856 (Rn) | 1537 | NP_004967 (Hs) (SEQ ID NO: 157), NP_032447 (Mm), NP_001106210 (Mm), NP_631963 (Rn), NP_036988 (Rn) |
| Shaw KCNC2 | voltage gated Potassium channel Delayed Rectifier Shaw Related member 2 | NM_153748 (Hs), NM_139136 (Hs), NM_139137 (Hs), NM_001025581 (Mm), NM_139217 (Rn), NM_139216 (Rn) | 1843 | NP_631875 (Hs) (SEQ ID NO: 158), NP_715624 (Hs) (SEQ ID NO: 159), NP_ 631874 (Hs) (SEQ ID NO: 160), NP_001020752 (Mm), NP_631962 (Rn), NP 631963 (Rn) |
| Shaw KCNC3 | voltage gated Potassium channel A-Type Shaw Related member 3 | NM_004977 (Hs), NM_008422 (Mm), NM_053997 (Rn) | 2278 | NP_004968 (Hs) (SEQ ID NO: 161), NP_032448 (Mm), NP_446449 (Rn) |
| Shaw KCNC4 | voltage gated Potassium channel A-Type Fast inactivation Shaw Related member 4 | NM_004978 (Hs) NM_145922 (Mm) | 1912 | NP_004969 (Hs) (SEQ ID NO: 162), NP_666034 (Mm), NP_001116248 (Rn) |
| Shal KCND1 | voltage gated Potassium channel A-Type Shal Related member 1 | NM_004979 (Hs), NM_008423 (Mm) NM_001105748 (Rn) | 1948 | NP_004970 (Hs) (SEQ ID NO: 163), NP_032449 (Mm), NP_001099218 (Rn) |
| Shal KCND2 | voltage gated Potassium channel A-Type Shal Related member 2 | NM_012281 (Hs), NM_019697 (Mm), NM_031730 (Rn) | 1894 | NP_036413 (Hs) (SEQ ID NO: 164), NP_062671 (Mm), NP_113918 (Rn) |
| Shal KCND3 | voltage gated Potassium channel A-Type Shal Related member 3 | NM_172198 (Hs), NM_004980 (Hs), NM_019931 (Mm), NM_031739 (Rn) | 1969 | NP_004971 (Hs) (SEQ ID NO: 165), NP_751948 (Hs) (SEQ ID NO: 166), NP_064315 (Mm), NP_113927 (Rn) |
| Modifier KCNF1 | voltage gated Potassium channel modifier of Kv2 | NM_002236 (Hs), NM_201531 (Mm), NM_001169104 (Rn) | 1486 | NP_002227 (Hs) (SEQ ID NO: 167), NP_963289 (Mm), NP_001162575 (Rn) |
| Modifier KCNG1 | voltage gated Potassium channel modifier/silencer of Kv2 | NM_002237 (Hs), NM_001081134 (Mm), NM_001106545 (Rn) | 1546 | NP_002228 (Hs) (SEQ ID NO: 168), NP_001074603 (Mm), NP_001100015 (Rn) |
| Modifier KCNG2 | voltage gated Potassium channel modifier/silencer of Kv2 | NM_012283 (Hs), NM_012283.1 (Hs), XM_140499 (Mm), NM_ 001107372 (Rn) | 1403 | NP_036415.1 (Hs) (SEQ ID NO: 169), NP_036415 (Hs) (SEQ ID NO: 170), XP_140499 (Mm), NP_001100842 (Rn) |
| Modifier KCNG3 | voltage gated Potassium channel modifier/silencer of Kv2 | NM_133329 (Hs), NM_172344 (Hs), NM_153512 (Mm), NM_001033957 (Rn), NM 133426 (Rn) | 1315 | NP_579875 (Hs) (SEQ ID NO: 171), NP_758847 (Hs) (SEQ ID NO: 172), NP_705732 (Mm), NP_596917 (Rn), NP 001029129 (Rn) |
| Modifier KCNG4 | voltage gated Potassium channel modifier/silencer of Kv2 | NM_172347 (Hs), NM_025734 (Mm), NM_001107435 (Rn) | 1564 | NP_758857 (Hs) (SEQ ID NO: 173), NP _080010 (Mm), NP 001100905 (Rn) |
| KCNQ1 | voltage gated Potassium channel Delayed Rectifier KQT-Like member 1 | NM_000218 (Hs), NM_181798 (Hs), NM_008434 (Mm), NM_032073 (Rn) | 2032 | NP _000209 (Hs) (SEQ ID NO: 174), NP_032460 (Mm), NP_114462 (Rn) |
| KCNQ2 | voltage gated Potassium channel Delayed Rectifier KQT-Like member 2 | NM_172107 (Hs), NM_010611 (Mm), NM_133322 (Rn) | 2620 | NP_742105 (Hs) (SEQ ID NO: 175), NP_034741 (Mm), NP_579856 (Rn) |
| KCNQ3 | voltage gated Potassium channel Delayed Rectifier KQT-Like member 3 | NM_004519 (Hs), NM_152923 (Mm), NM _ 031597 (Rn) | 2620 | NP_004510 (Hs) (SEQ ID NO: 176), NP_690887 (Mm), NP_113785 (Rn) |
| KCNQ4 | voltage gated Potassium channel Delayed Rectifier KQT-Like member 4 | NM_172163.1 (Hs), NM_004700 (Hs), NM_001081142 (Mm), XM_ 233477 (Rn) | 2089 | NP_751895 (Hs) (SEQ ID NO: 177), NP_004691 (Hs) (SEQ ID NO: 178), NP_001074611 (Mm), XP_233477 (Rn) |
| KCNQ5 | voltage gated Potassium channel modifier/silencer of Kv2 | NM_019842 (Hs), NM_023872 (MM), XM_001071249 (Rn) | 2803 | NP_062816 (Hs) (SEQ ID NO: 179), NP_076361 (Mm), XP_001071249 (Rn) |
| KCNV1 | voltage gated Potassium channel modifier/silencer of Kv2 | NM 014379 (Hs), NM_026200 (Mm), NM_021697 (Rn) | 1507 | NP_055194 (Hs) (SEQ ID NO: 180), NP_080476 (Mm), NP_067729 (Rn) |
| KCNV2 | voltage gated Potassium channel modifier/silencer of Kv2 | NM_133497 (Hs), NM_183179 (Mm), NM_001106370 (Rn) | 1642 | NP_598004 (Hs) (SEQ ID NO: 181), NP_899002 (Mm), NP_001099840 |
| KCNS1 | voltage gated Potassium channel modifier/silencer of Kv2 | NM_002251 (Hs), NM_008435 (Mm), NM_053954 (Rn) | 1585 | NP_002242 (Hs) (SEQ ID NO: 182), NP_032461 (Mm), NP_446406 (Rn) |
| KCNS2 | voltage gated Potassium channel modifier/silencer of Kv2 | NM_020697 (Hs), NM_181317 (Mm), NM_023966 (Rn) | 1438 | NP_065748 (Hs) (SEQ ID NO: 183), NP_851834 (Mm), NP_076456 (Rn) |
| KCNS3 | voltage gated Potassium channel modifier/silencer of Kv2 | NM_002252 (Hs), NM_173417 (Mm), NM_031778 (Rn) | 1477 | NP_002243 (Hs) (SEQ ID NO: 184), NP_775593 (Mm), NP_113966 (Rn) |
| KCNH1 | voltage gated Potassium channel Delayed Rectifier Ether-A-Gogo (eag1) | NM_172362 (Hs), NM_010600 (Mm), NM_031742 (Rn) | 2974 | NP_758872 (Hs) (SEQ ID NO: 185), NP_034730 (Mm), NP_113930 (Rn) |
| KCNH5 | voltage gated Potassium channel Outward-Rectifying Ether-A-Gogo (eag2) | NM_172375 (Hs), NM_172376 (Hs), NM_139318 (Hs), NM_172805 (Mm), NM_133610 (Rn) | 2971 | NP_758963 (Hs) (SEQ ID NO: 186), NP_758964 (Hs) (SEQ ID NO: 187), NP _647479 (Hs), NP_ _766393 (Mm), NP 598294 (Rn) |
| KCNH2 | voltage gated Potassium channel Inwardly- Rectifying Ether-A-Gogo (erg1 or HERG) | NM_172056 (Hs), NM_000238 (Hs), NM_172057 (Hs), NM 013569 (Mm), NM_053949 (Rn) | 3484 | NP_000229 (Hs) (SEQ ID NO: 188), NP_742053 (Hs) (SEQ ID NO: 189), NP_742054 (Hs) (SEQ ID NO: 190), NP_ 038597 (Mm), NP 446401 (Rn) |
| KCNH6 | voltage gated Potassium channel Inwardly- Rectifying Ether-A-Gogo (erg2) | NM_030779 (Hs), NM_173092 (Hs), NM_001037712 (Mm), NM_053937 (Rn) | 2989 | NP_110406 (Hs) (SEQ ID NO: 191), NP_775115 (Hs) (SEQ ID NO: 192), NP_001032801 (Mm), NP_446389 (Rn) |
| KCNH7 | voltage gated Potassium channel Inwardly- Rectifying Ether-A-Gogo (erg3) | NM_173162 (Hs), NM_033272 (Hs), NM_133207 (Mm), NM_131912 (Rn) | 3595 | NP_775185 (Hs) (SEQ ID NO: 193), NP_150375 (Hs) (SEQ ID NO: 194), NP_573470 (Mm), NP_571987 (Rn) |
| KCNH8 | voltage gated Potassium channel Inwardly- Rectifying Ether-A-Gogo (elk1) | NM_144633 (Hs), NM_001031811 (Mm), NM_145095 (Rn) | 3328 | NP_653234 (Hs) (SEQ ID NO: 195), NP_001026981 (Mm), NP_659563 (Rn) |
| KCNH3 | voltage gated Potassium channel Inwardly- Rectifying Ether-A-Gogo (elk2) | NM_012284 (Hs), NM_010601 (Mm), NM_(Rn) | 017108 3259 | NP_036416 (Hs) (SEQ ID NO: 196), NP_034731 (Mm), NP_058804 (Rn) |
| KCNH4 | voltage gated Potassium channel Inwardly- Rectifying Ether-A-Gogo (elk1) | NM_012285 (Hs), NM_001081194 (Mm), NM_053630 (Rn) | 3058 | NP_036417 (Hs) (SEQ ID NO: 197), NP _001074663 (Mm), NP_446082 (Rn) |

| **Canaux potassiques activés par le calcium intracellulaire** | | | | |
|---|---|---|---|---|
| KCNMA1 | large conductance calcium-activated Potassium channel Slo1 | NM_001014797 (Hs), NM_010610 (Mm), NM_031828 (Rn) | 3550 | NP_001014797 (Hs) (SEQ ID NO: 198), NP_034740 (Mm), NP_114016 (Rn) |
| KCNN1 | small conductance calcium-activated Potassium channel SK1 | NM_002248 (Hs), NM_032397 (Mm), NM_019313 (Rn) | 1636 | NP_002239 (Hs) (SEQ ID NO: 199), NP_115773 (Mm), NP_062186 (Rn) |
| KCNN2 | small conductance calcium-activated Potassium channel SK2 | NM_170775 (Hs), NM_021614 (Hs), NM_080465 (Mm), NM_019314 (Rn) | 1744 | NP_740721 (Hs) (SEQ ID NO: 200), NP_067627 (Hs) (SEQ ID NO: 201), NP_536713 (Mm), NP_062187 (Rn) |
| KCNN3 | small conductance calcium-activated Potassium channel SK3 | NM_002249 (Hs), NM_170782 (Hs), NM_080466 (Mm), NM_019315 (Rn) | 2197 | NP_740752 (Hs) (SEQ ID NO: 202), NP_002240 (Hs) (SEQ ID NO: 203), NP_536714 (Mm), NP_062188 (Rn) |
| KCNN4 | intermediate conductance calcium-activated Potassium channel SK4 | NM_002250 (Hs), NM 008433 (Mm), (Rn) | NM_023021 1285 | NP_002241 (Hs) (SEQ ID NO: 204), NP_032459 (Mm), NP_075410 (Rn) |
| KCNT1 | sodium activated Potassium channel Slo2.2 (Slack) | NM_020822 (Hs), NM_175462 (Mm), NM_021853 (Rn) | 3775 | NP_065873 (Hs) (SEQ ID NO: 205), NP_780671 (Mm), NP_068625 (Rn) |
| KCNT2 | sodium activated Potassium channel Slo2.1 (Slick) | NM_198503 (Hs), NM 001081027 (Mm), NM_198762 (Rn) | 3409 | NP_940905 (Hs) (SEQ ID NO: 206), NP_001074496 (Mm), NP_942057 (Rn) |
| KCNU1 | sodium activated Potassium channel Slo3 | NM_001031836 (Hs), NM_008432 (Mm) | 3454 | NP_001027006 (Hs) (SEQ ID NO: 207), NP_032458 (Mm) |

| **Canaux potassiques de la rectification entrante** | | | | |
|---|---|---|---|---|
| KCNJ1 | Inwardly rectifying potassium channel Romk1 | NM_000220 (Hs), NM_153764 (Hs), NM_019659 (Mm), NM_ 017023 (Rn) | 1180 | NP_722448 (Hs) (SEQ ID NO: 208), NP_000211 (Hs) (SEQ ID NO: 209), NP_062633 (Mm), NP 058719 (Rn) |
| KCNJ2 | Inwardly rectifying potassium channel IRK1 | NM_000891 (Hs), NM_008425 (Mm), NM_017296 (Rn) | 1285 | NP_000882 (Hs) (SEQ ID NO: 210), NP_032451 (Mm), NP_058992 (Rn) |
| KCNJ12 | Inwardly rectifying potassium channel IRK2 | NM_021012 (Hs), NM_010603 (Mm), NM_053981 (Rn) | 1306 | NP_066292 (Hs) (SEQ ID NO: 211), NP_034733 (Mm), NP_446433 (Rn) |
| KCNJ4 | Inwardly rectifying potassium channel IRK3 | NM_152868 (Hs), NM_008427 (Mm), NM_053870 (Rn) | 1342 | NP_690607 (Hs) (SEQ ID NO: 212), NP_032453 (Mm), NP_446322 (Rn) |
| KCNJ14 | Inwardly rectifying potassium channel IRK4 | NM_170720 (Hs), NM_145963 (Mm), NM_170718 (Rn) | 1312 | NP_733838 (Hs) (SEQ ID NO: 213), NP_666075 (Mm), NP_733836 (Rn) |
| KCNJ3 | Inwardly rectifying potassium channel GIRK1 | NM_002239 (Hs), NM_008426 (Mm), NM_031610 (Rn) | 1510 | NP_002230 (Hs) (SEQ ID NO: 214), NP_032452 (Mm), NP_113798 (Rn) |
| KCNJ6 | Inwardly rectifying potassium channel GIRK2 | NM_002240 (Hs), NM_010606 (Mm), NM_001025590 (Mm), NM_001025585 (Mm), NM_001025584 (Mm), NM 013192 | 1273 | NP_002231 (Hs) (SEQ ID NO: 215), NP_001020756 (Mm), NP_001020755 (Mm), NP_034736 (Mm), NP_001020761 (Mm), NP_037324 (Rn) |
| KCNJ9 | Inwardly rectifying potassium channel GIRK3 | NM_004983 (Hs), NM_008429 (Mm), NM_ 053834 (Rn) | 1186 | NP_004974 (Hs) (SEQ ID NO: 216), NP_032455 (Mm), NP_446286 (Rn) |
| KCNJ5 | Inwardly rectifying potassium channel GIRK4 | NM_000890 (Hs), NM_010605 (Mm), NM_017297 (Rn) | 1264 | NP_000881 (Hs) (SEQ ID NO: 217), NP_034735 (Mm), NP_058993 (Rn) |
| KCNJ10 | Inwardly rectifying potassium channel BIR10 | NM_002241 (Hs), NM_001039484 (Mm), NM_031602 (Rn) | 1144 | NP_002232 (Hs) (SEQ ID NO: 218), NP_001034573 (Mm), NP_113790 (Rn) |
| KCNJ15 | Inwardly rectifying potassium channel IRKK (Kir1.3) | NM_170736 (Hs), NM_001039057 (Mm), NM_001039056 (Mm), NM_ 133321 (Rn) | 1129 | NP_733932 (Hs) (SEQ ID NO: 219), NP_001034145 (Mm), NP_001034146 (Mm), NP_579855 (Rn) |
| KCNJ16 | Inwardly rectifying potassium channel BIR9 | NM_018658 (Hs), NM_010604 (Mm), NM_053314 (Rn) | 1261 | NP_061128 (Hs) (SEQ ID NO: 220), NP_034734 (Mm), NP_445766 (Rn) |
| KCNJ8 | ATP-sensitive potassium channel K-ATP1 | NM_004982 (Hs), NM_008428 (Mm), NM_017099 (Rn) | 1279 | NP_004973 (Hs) (SEQ ID NO: 221), NP_032454 (Mm), NP_058795 (Rn) |
| KCNJ11 | ATP-sensitive potassium channel BIR | NM_000525 (Hs), NM_010602 (Mm), NM_031358 (Rn) | 1177 | NP_000516 (Hs) (SEQ ID NO: 222), NP_034732 (Mm), NP_112648 (Rn) |
| KCNJ13 | ATP-sensitive potassium channel (Kir1.4) | NM_002242 (Hs), NM_053608 (Rn) | 1084 | NP_002233 (Hs) (SEQ ID NO: 223), NP_446060 (Rn) |

| **Canaux potassiques à 2 domaines P** | | | | |
|---|---|---|---|---|
| KCNK1 | two pore domain potasium channel TWIK1 | NM_002245 (Hs), NM_008430 (Mm), NM_021688 (Rn) | 1008 | NP_002236 (Hs) (SEQ ID NO: 224), NP_032456 (Mm), NP_067720 (Rn) |
| KCNK2 | two pore domain potasium channel TREK1 | NM_001017424 (Hs), NM_010607 (Mm), NM_172042 | 1278 | NP_001017424 (Hs) (SEQ ID NO: 225), NP_ 034737 (Mm), NP_742038 (Rn) |
| KCNK3 | two pore domain potasium channel TASK1 | NM_002246 (Hs), NM_010608 (Mm), NM_033376 (Rn) | 1182 | NP_002237 (Hs) (SEQ ID NO: 226), NP_034738 (Mm), NP_203694 (Rn) |
| KCNK4 | two pore domain potasium channel TRAAK | NM_033310 (Hs), NM_008431 (Mm), NM_053804 (Rn) | 1179 | NP_201567 (Hs) (SEQ ID NO: 227), NP_032457 (Mm), NP_446256 (Rn) |
| KCNK5 | two pore domain potasium channel TASK2 | NM_003740 (Hs), NM_021542 (Mm), NM_001039516 (Rn) | 1497 | NP_003731 (Hs) (SEQ ID NO: 228), NP_067517 (Mm), NP _001034605 (Rn) |
| KCNK6 | two pore domain potasium channel TWIK2 | NM_004823 (Hs), NM_001033525 (Mm), NM_053806 (Rn) | 939 | NP_004814 (Hs) (SEQ ID NO: 229), NP_001028697 (Mm), NP_446258 (Rn) |
| KCNK7 | two pore domain potasium channel member 7 | NM_033347 (Hs), NM_010609 (Mm) | 921 | NP_203133 (Hs) (SEQ ID NO: 230), NP_034739 (Mm) |
| KCNK9 | two pore domain potasium channel TASK3 | NM_016601 (Hs), NM_001033876 (Mm), NM_053405 (Rn) | 1122 | NP_057685 (Hs) (SEQ ID NO: 231), NP_001029048 (Mm), NP_445857 (Rn) |
| KCNK10 | two pore domain potasium channel TREK2 | NM_021161 (Hs), NM_029911 (Mm), NM_023096 (Rn) | 1614 | NP_066984 (Hs) (SEQ ID NO: 232), NP_084187 (Mm), NP_075584 (Rn) |
| KCNK12 | two pore domain potasium channel THIK2 | NM_022055 (Hs), NM_199251 (Mm), NM_022292 (Rn) | 1290 | NP_071338 (Hs) (SEQ ID NO: 233), NP_954859 (Mm), NP_071628 (Rn) |
| KCNK13 | two pore domain potasium channel THIK1 | NM_022054 (Hs), NM_146037 (Mm), NM_022293 (Rn) | 1224 | NP_071337 (Hs) (SEQ ID NO: 234), NP_666149 (Mm), NP_071629 (Rn) |
| KCNK15 | two pore domain potasium channel TASK5 | NM_022358 (Hs), NM_001030292 (Mm), NM_130813 (Rn) | 990 | NP_071753 (Hs) (SEQ ID NO: 235), NP_001025463 (Mm), NP_570826 (Rn) |
| KCNK16 | two pore domain potasium channel TALK1 | NM_032115 (Hs), NM_029006 (Mm) | 930 | NP_115491 (Hs) (SEQ ID NO: 236), NP_083282 (Mm) |
| KCNK17 | two pore domain potasium channel TASK4-TALK2 | NM_031460 (Hs) | 996 | NP_113648 (Hs) (SEQ ID NO: 237) |
| KCNK18 | two pore domain potasium channel TRESK1/TRESK2 | NM_181840 (Hs), NM_207261 (Mm), NM_001003820 (Rn) | 1152 | NP_862823 (Hs) (SEQ ID NO: 238), NP_997144 (Mm), NP_001003820 |

| **Canaux ioniques activés par les nucléotides** | | | | |
|---|---|---|---|---|
| CNGA1 | Cyclic nucleotide gated cation channel Alpha 1 | NM_000087 (Hs), NM_007723 (Mm), NM_053497 (Rn) | 2073 | NP_000078 (Hs) (SEQ ID NO: 239), NP_031749 (Mm), NP_445949 (Rn) |
| CNGA2 | Cyclic nucleotide gated cation channel Alpha 2 | NM_005140 (Hs), NM_007724 (Mm), NM_012928 (Rn) | 1995 | NP_005131 (Hs) (SEQ ID NO: 240), NP_031750 (Mm), NP_037060 (Rn) |
| CNGA3 | Cyclic nucleotide gated cation channel Alpha 3 | NM_001298 (Hs), NM_009918 (Mm), NM_053495 (Rn) | 2086 | NP_001073347 (Hs) (SEQ ID NO: 241), NP 034048 (Mm), NP 445947 |
| CNGA4 | Cyclic nucleotide gated cation channel Alpha 4 | NM_001037329 (Hs), NM_001033317 (Mm), NM_053496 (Rn) | 1731 | NP_001032406 (Hs) (SEQ ID NO: 242), NP_001028489 (Mm), NP_445948 (Rn) |
| CNGB1 | Cyclic nucleotide gated cation channel Betal | NM_001297 (Hs), NM_031809 (Rn) | 3763 | NP_001288 (Hs) (SEQ ID NO: 243), XP_001476248 (Mm), NP_113997 |
| CNGB3 | Cyclic nucleotide gated cation channel Beta3 | NM_019098 (Hs), NM_013927 (Mm) | 2434 | NP_061971 (Hs) (SEQ ID NO: 244), NP_038955 (Mm) |
| HCN1 | hyperpolarization Cyclic nucleotide gated cation channel member 1 | NM_021072 (Hs), NM_010408 (Mm), NM_053375 (Rn) | 2674 | NP_066550 (Hs) (SEQ ID NO: 245), NP_034538 (Mm), NP_445827 (Rn) |
| HCN2 | hyperpolarization Cyclic nucleotide gated cation channel member 2 | NM_001194 (Hs), NM_008226 (Mm), NM_053684 (Rn) | 2671 | NP 001185 (Hs) (SEQ ID NO: 246), NP_032252 (Mm), NP_446136 (Rn) |
| HCN3 | hyperpolarization Cyclic nucleotide gated cation channel member 3 | NM_020897 (Hs), NM_008227 (Mm), NM_053685 (Rn) | 2326 | NP_065948 (Hs) (SEQ ID NO: 247), NP_032253 (Mm), NP_446137 (Rn) |
| HCN4 | hyperpolarization Cyclic nucleotide gated cation channel member 4 | NM_005477 (Hs), NM_001081192 (Mm), NM_ 021658 (Rn) | 3613 | NP_005468 (Hs) (SEQ ID NO: 248), NP_001074661 (Mm), NP_067690 (Rn) |

| **Canaux de type TRP** | | | | |
|---|---|---|---|---|
| TRPC1 | transient receptor potential (TRP) non selective cation channel C member 1 | NM_003304 (Hs), NM_011643 (Mm), NM_053558 (Rn) | 2284 | NP_003295 (Hs) (SEQ ID NO: 249), NP_035773 (Mm), NP_446010 (Rn) |
| | | | | |
| TRPC3 | diacyglycerol (DAG) Ca2+ activated TRP channel C | NM_003305 (Hs), NM_019510 (Mm), NM_021771 (Rn) | 2548 | NP_003296 (Hs) (SEQ ID NO: 250), NP_062383 (Mm), NP_068539 (Rn) |
| TRPC4 | TRP receptor operated channel | NM_016179 (Hs), NM_016984 (Mm), NM_080396 (Rn), NM_001083115 | 2935 | NP_057263 (Hs) (SEQ ID NO: 251), NP_058680 (Mm), NP_001076584 (Rn), NP_536321 (Rn) |
| TRPC5 | TRP receptor operated channel | NM 012471 (Hs), NM_009428 (Mm), NM_080898 (Rn) | 2923 | NP_036603 (Hs) (SEQ ID NO: 252), NP_033454 (Mm), NP_543174 (Rn) |
| TRPC6 | DAG activated TRP channel | NM_004621 (Hs), NM_013838 (Mm), NM_053559 (Rn) | 2800 | NP_004612 (Hs) (SEQ ID NO: 253), NP_038866 (Mm), NP_446011 (Rn) |
| TRPC7 | Cardiac TRP channel | NM_020389 (Hs), NM_012035 (Mm), XM_225159 (Rn) | 2593 | NP_065122 (Hs) (SEQ ID NO: 254), NP_036165 (Mm), XP_001067646 |
| TRPV1 | Vanilloid (capsaicin) receptor and noxious thermosensor channel | NM_018727 (Hs), NM_001001445 (Mm), NM_031982 (Rn) | 2524 | NP_061197 (Hs) (SEQ ID NO: 255), NP_001001445 (Mm), NP_114188 (Rn) |
| TRPV2 | noxious heat thermosensor channel | NM_016113 (Hs), NM_011706 (Mm), NM_017207 (Rn) | 2299 | NP_057197 (Hs) (SEQ ID NO: 256), NP_035836 (Mm), NP_058903 (Rn) |
| TRPV3 | warmth sensor channel | NM_145068 (Hs), NM_145099 (Mm), NM_001025757 (Rn) | 2380 | NP 659505 (Hs) (SEQ ID NO: 257), NP_659567 (Mm), NP_001020928 |
| TRPV4 | Osmosensor channel | NM_021625 (Hs), NM_022017 (Mm), NM_023970 (Rn) | 2620 | NP_ 067638 (Hs) (SEQ ID NO: 258), NP_071300 (Mm), NP_076460 (Rn) |
| TRPM1 | putative melastatin TRP channel | NM_002420 (Hs), NM_018752 (Mm), NM_001037734 (Rn) | 4816 | NP_002411 (Hs) (SEQ ID NO: 259), NP_061222 (Mm), NP_001032823 |
| TRPM2 | nucleotide sensing TRP channel | NM_003307 (Hs), NM_138301 (Mm), NM_001011559 (Rn) | 4513 | NP_003298 (Hs) (SEQ ID NO: 260), NP_612174 (Mm), NP_001011559 (Rn) |
| TRPM3 | Melastatin related TRP channel | NM_020952 (Hs), NM_001035239 (Mm), XM_219902 (Rn) | 4669 | NP_066003 (Hs) (SEQ ID NO: 261), NP_001030319 (Mm), XP_219902 (Rn) |
| TRPM4 | Ca2+ activated Na+ TRP channel | (Mm), NM_001136229 (Rn) NM_017636 (Hs), NM_175130 | 3646 | NP_060106 (Hs) (SEQ ID NO: 262), NP_780339 (Mm), NP _001129701 (Rn) |
| TRPM5 | Nonselective, monovalent cation TRP channel | NM_014555 (Hs), NM_020277 (Mm), XM_344979 (Rn) | 3502 | NP_055370 (Hs) (SEQ ID NO: 263), NP_064673 (Mm), XP_344980 (Rn) |
| TRPM6 | Channel kinase TRP channel | NM_017662 (Hs), NM_153417 (Mm), XM_219747 (Rn) | 6073 | NP_060132 (Hs) (SEQ ID NO: 264), NP_700466 (Mm), XP_219747 (Rn) |
| TRPM7 | Kinase TRP channel | NM_017672 (Hs), NM_021450 (Mm), XM_001056331 (Rn) | 5602 | NP_060142 (Hs) (SEQ ID NO: 265), NP_067425 (Mm), XP_001056331 (Rn) |
| TRPM8 | Cooling and menthol-sensing TRP channel | NM_024080 (Hs), NM_134252 (Mm), NM_134371 (Rn) | 3319 | NP_076985 (Hs) (SEQ ID NO: 266), NP _599013 (Mm), NP _599198 (Rn) |
| TRPA1 | Nonselective cation TRP channel | NM_007332 (Hs), NM_177781 (Mm), NM_207608 (Rn) | 3364 | NP_015628 (Hs) (SEQ ID NO: 267), NP_808449 (Mm), NP_997491 (Rn) |
| TRPP1 | Nonselective cation TRP channel PKD2 | NM_000297 (Hs), NM_008861 (Mm), XM_573552 (Rn) | 2908 | NP_000288 (Hs) (SEQ ID NO: 268), NP_032887 (Mm), XP_573552 (Rn) |
| TRPP2 | Nonselective cation TRP channel PKD2L1 | NM_016112 (Hs), NM_181422 (Mm), NM_001106352 (Rn) | 2422 | NP_057196 (Hs) (SEQ ID NO: 269), NP_852087 (Mm), NP_001099822 |
| TRPP3 | Nonselective cation TRP channel PKD2L2 | NM_014386 (Hs), NM_016927 (Mm), NM_001106156 (Rn) | 1843 | NP_055201 (Hs) (SEQ ID NO: 270), NP_058623 (Mm), NP_001099626 |
| TRPML1 | Nonselective cation TRP channel | NM_020533 (Hs) NM_053177 (Mm), NM_001105903 (Rn) | 1747 | NP_065394 (Hs) (SEQ ID NO: 271), NP_444407 (Mm), NP_001099373 |
| TRPML2 | Nonselective cation TRP channel | NM_153259 (Hs), NM_001005846 (Mm), NM_026656 (Mm), NM_001039005 (Rn) | 1702 | NP_694991 (Hs) (SEQ ID NO: 272), NP_001005846 (Mm), NP_080932 (Mm), NP_001034094 (Rn) |
| TRPML3 | Nonselective cation TRP channel | NM_018298 (Hs), NM_134160 (Mm), NM_001012059 (Rn) | 1663 | NP_060768 (Hs) (SEQ ID NO: 273), (Mm), NP_001012059 |

| **Canaux chlores** | | | | |
|---|---|---|---|---|
| CLC1 | chloride channel 1 | NM_000083.2 | 2968 | NP_000074.2 (SEQ ID NO: 274) |
| CLC2 | chloride channel 2 | NM_004366.4 | 2698 | NP_004357.3 (SEQ ID NO: 275) |
| CLC3 | chloride channel 3 | NM_1738723 | 2602 | NP_776297.2 (SEQ ID NO: 276) |
| CLC4 | chloride channel 4 | NP_001821.2 | 2284 | NP_001821.2 (SEQ ID NO: 277) |
| CLC5 | chloride channel 5 | NM_000084.2 | 2242 | NP_000075.1 (SEQ ID NO: 278) |
| CLC6 | chloride channel 6 | NM_001286.2 | 2611 | NP_001277.1 (SEQ ID NO: 279) |
| CLC7 | chloride channel 7 | NM_001287.4 | 2419 | NP_001278.1 (SEQ ID NO: 280) |

| **Canaux cationiques sensibles à l'acidité** | | | | |
|---|---|---|---|---|
| ACCN1 | amiloride-sensitive cation channel 1 | NM_183377.1 | 1696 | NP_899233.1 (SEQ ID NO: 281) |
| ACCN2 | amiloride-sensitive cation channel 2 | NM_020039.2 | 1726 | NP_064423.2 (SEQ ID NO: 282) |
| ACCN3 | amiloride-sensitive cation channel 3 | NM_020322.2 | 1600 | NP_064718.1 (SEQ ID NO: 283) |
| ACCN4 | amiloride-sensitive cation channel 4 | NM_018674.4 | 2005 | NP_061144.3 (SEQ ID NO: 284) |

| **Sous-unités auxiliaires des canaux sodiques** | | | | |
|---|---|---|---|---|
| SCN1B | voltage gated sodium channel type I b subunit | NM_001037.4 | 658 | NP_001028.1 (SEQ ID NO: 285) |
| SCN2B | voltage gated sodium channel type 2 b subunit | NM_004588.4 | 652 | NP_004579.1 (SEQ ID NO: 286) |
| SCN3B | voltage gated sodium channel type 3 b subunit | NM_018400.3 | 652 | NP_060870.1 (SEQ ID NO: 287) |
| SCN4B | voltage gated sodium channel type 4 b subunit | NM_174934.3 | 694 | NP_777594.1 (SEQ ID NO: 288) |

| **Sous-unités auxiliaires des canaux calciques** | | | | |
|---|---|---|---|---|
| CACNB1 | voltage gated calcium channel b1 subunit | NM_000723.4 | 1798 | NP_000714.3 (SEQ ID NO: 289) |
| CACNB2 | voltage gated calcium channel b2 subunit | NM_000724.3 | 1822 | NP_000715.2 (SEQ ID NO: 290) |
| CACNB3 | voltage gated calcium channel b3 subunit | NM_000725.3 | 1459 | NP_000716.2 (SEQ ID NO: 291) |
| CACNB4 | voltage gated calcium channel b4 subunit | NM_000726.3 | 1465 | NP_000717.2 (SEQ ID NO: 292) |
| CACNA2 | voltage gated calcium channel a2d subunit | NM_000722.2 (HS), NM_001110843.1 (Mm) NM_012919 (RN) | 3277 | NP_000713.2 (HS) (SEQ ID NO: 293), NP_001104313.1 (Mm), NP_037051 (RN) |
| CACNG2 | voltage gated calcium channel g subunit | NM_006078.3 (HS), NM_007583.2 (Mm) | 973 | NP_006069.1 (HS) (SEQ ID NO: 294), NP_031609.1 (Mm) |

| **AUTRES** | | | | |
|---|---|---|---|---|
| ABCC8 | ATP-binding cassette, sub-family C SURI | NM_000352.3 (HS) NM_011510.3 (Mm) | | NP_000343.2 (HS) (SEQ ID NO: 295), NP_035640.2 (Mm) |
| ABCC9 | SUR2 | NM_020297.2 | | NP_064693.2 (SEQ ID NO: 296) |
| LGi1 | LGi1 | NM_005097.2 | | NP_005088.1 (SEQ ID NO: 297) |
| CNTNAP2 | CASPR2 | NM_014141.5 | | NP_054860.1 (SEQ ID NO: 298) |
| KCNAB1 | Kv b1 | NM_172160.2 | | NP_751892.1 (SEQ ID NO: 299) |
| KCNAB2 | Kv b2 | NM_003636.3 | | NP_003627.1 (SEQ ID NO: 300) |
| KCNAB3 | Kv b3 | NM_004732.2 | | NP_004723.2 (SEQ ID NO: 301) |
| KCNMB1 | BK b1 | NM_004137.2 | | NP_004128.1 (SEQ ID NO: 302) |
| KCNMB2 | BK b2 | NM_005832.3 | | NP_005823.1 (SEQ ID NO: 303) |
| KCNMB3 | BK b3 | NM_171828.1 | | NP_741979.1 (SEQ ID NO: 304) |
| KCNMB4 | BK b4 | NM_014505.5 | | NP_055320.4 (SEQ ID NO: 305) |
| AQP1 | AQP1 | NM_198098.2 | | NP_932766.1 (SEQ ID NO: 306) |
| AQP2 | AQP2 | NM_000486.5 | | NP_000477.1 (SEQ ID NO: 307) |
| AQP3 | AQP3 | NM_004925.4 | | NP_004916.1 (SEQ ID NO: 308) |
| AQP4 | AQP4 | NM_001650.4 | | NP_001641.1 (SEQ ID NO: 309) |
| GJB1 | Connexin 32 | NM_000166.5, NM_000165.3 | | NP_000157.1 (SEQ ID NO: 310) |
| GJA1 | Connexin 43 | NM_000165.3 | | NP_000156.1 (SEQ ID NO: 311) |

| | | | | |
|---|---|---|---|---|
| Hs : Homo sapiens (humain) Mm : Mus musculus (souris) Rn : Rattus norvegicus (rat) | | | | |

Dans un mode de réalisation particulier de l'invention, la protéine membranaire d'intérêt est choisie parmi un récepteur du GABA, typiquement un récepteur GABA_{B} (GABA_{B}R1 ou GABA_{B}R2 par exemple) ; un récepteur du glutamate, typiquement un récepteur alpha-amino-3-hydroxy-5-méthylisoazole-4-propionique acid (AMPA), de préférence la sous-unité GluR3 du récepteur du glutamate (SEQ ID NO :73 ou SEQ ID NO :74) ou un récepteur du glutamate activé par la N-méthyl-D-aspartate (NMDA), de préférence la sous-unité NMDAR1 du récepteur humain du glutamate (SEQ ID NO :62) ; l'aquaporine 2 (SEQ ID NO :307); l'aquaporine 4 (SEQ ID NO :309); un canal potassique ; un canal calcique ; un récepteur de l'acétylcholine, de préférence la sous-unité α3 (SEQ ID NO :9) du récepteur humain de l'acétylcholine nicotinique (ACHα3) ; une protéine associée aux canaux potassiques dépendant du voltage, de préférence CASPR2 (SEQ ID NO :298) ou LGI1 (SEQ ID NO :297); un récepteur Glycine; la connexine 32 (SEQ ID NO :310), et la connexine 43 (SEQ ID NO :311).

Un autre objet de l'invention concerne une puce comprenant au moins 20 protéines d'intérêt différentes, par exemple au moins 50, typiquement au moins 100 protéines d'intérêt différentes, de préférence entre environ 20 et environ 400 protéines d'intérêt différentes, par exemple environ 250 ou environ 300 protéines d'intérêt différentes, ou entre environ 20 et environ 100 protéines d'intérêt différentes, par exemple environ 50 protéines d'intérêt différentes, chaque protéine d'intérêt étant éventuellement associée à une ou à plusieurs sous-unités auxiliaires. Ces protéines d'intérêt sont de préférence des protéines membranaires choisies parmi les protéines dont les séquences (de préférence les séquences des protéines humaines) sont identifiées dans le tableau 2.

Les inventeurs ont en particulier mis au point des puces comprenant des protéines membranaires correspondant à des protéines de type protéines canal ionique, protéines transporteurs et/ou protéines récepteur membranaire régulant l'activité d'une protéine canal ionique ou d'une protéine transporteur. Ces protéines sont codées par les séquences, de préférence par les séquences identifiées par un numéro SEQ ID, encore plus préférentiellement par les séquences humaines identifiées par un numéro SEQ ID, référencées dans le tableau 2.
Pour fabriquer ces puces, les inventeurs ont sans *a priori* amplifié par PCR un grand nombre de sous-unités obtenues à partir d'ADNc de cerveau humain (voir partie expérimentale). Les produits ont été sous-clonés dans un vecteur d'expression et transfectés dans des cellules en culture en plaque multi-puits, chaque puits contenant des cellules exprimant un seul type de protéine membranaire ou complexe protéique membranaire. Une même séquence *tag* introduite dans chaque canal permet, conformément aux connaissances de l'homme du métier, de mesurer et normaliser le niveau d'expression des protéines ou complexes protéiques. Les protéines et complexes protéiques de ces cellules sont solubilisées dans un tampon contenant un détergent non dénaturant. Ce solubilisat est ensuite typiquement déposé sous la forme de spot sur un support, de préférence sur un support solide, par exemple sur une membrane solide. Typiquement, différentes quantités de solubilisat, ainsi que de préférence un ou plusieurs contrôles négatifs et positifs, sont déposés sur le même support afin de vérifier la spécificité des signaux obtenus. La lecture de la puce (détection des complexes antigènes/anticorps (Ag/Ac)) peut être réalisée par immunofluorescence, immunoluminescence, ou colorimétrie à l'aide de l'une des techniques connues de l'homme du métier.

Ainsi, un objet particulier de l'invention concerne une puce telle que décrite dans le présent texte dont ladite ou lesdites protéines d'intérêt sont marquées à l'aide d'une même séquence peptidique (étiquette ou tag), reconnue par un ou plusieurs anticorps de détection connus de l'homme du métier, tel que par exemple l'anticorps V5 qui reconnaît la séquence GKPIPNPLLGLDST (SEQ ID NO :312). D'autres étiquettes utilisables dans le contexte de la présente invention sont par exemple les étiquettes (ou tag) T7, Flag, Ha, myc et His (hexahistidine).

Dans un mode de réalisation préféré, la puce à protéines selon l'invention comprend au moins un contrôle positif et/ou un contrôle négatif, typiquement un contrôle positif et un contrôle négatif, afin de vérifier la spécificité des signaux obtenus. Le contrôle négatif est typiquement constitué par un extrait cellulaire ne comprenant pas la protéine d'intérêt. Le contrôle positif est typiquement constitué par des immunoglobulines humaines, par exemple des IgG, et permet de valider la capacité de la puce à protéines selon l'invention à détecter la présence d'autoanticorps capables de se lier aux protéines d'intérêt au sein d'un échantillon biologique à tester.

Les puces utilisées dans le cadre de l'invention comprennent un support susceptible de fixer des protéines solubilisées, typiquement un support solide (ou semi-solide), de préférence choisi parmi une membrane, typiquement une membrane de nitrocellulose ou de nylon ; du verre ; et un polymère capable d'absorber les complexes immuns, typiquement du plastique (par exemple du PVDF).
Un tel support peut être traité pour améliorer ses propriétés de fixation des extraits cellulaires à l'aide de produits et selon des méthodes connus de l'homme du métier. Le support peut par exemple être revêtu d'une couche de composés organosilicés (silane) et/ou être fonctionnalisé à l'aide de glycosaminoglycanes (GAG).

Un autre objet de l'invention concerne par ailleurs un kit comprenant une puce telle que décrite précédemment, et, éventuellement, un ou plusieurs réactifs choisis de préférence parmi un tampon de blocage des sites non spécifiques, un tampon permettant l'association entre anticorps et antigènes (Ac/Ag), un tampon de lavage, un ou plusieurs anticorps secondaire, un produit ou plusieurs produits de détection dudit ou desdits anticorps secondaire, une solution standard permettant de préparer une courbe d'étalonnage, et des instructions d'utilisation, typiquement des instructions d'utilisation de l'un, de plusieurs ou de l'ensemble desdits éléments du kit.
Un tel kit pourra typiquement être utilisé dans le criblage de nouvelles cibles d'intérêt, par exemple de cibles impliquées dans la survenue d'une maladie autoimmune, en particulier d'une maladie affectant le système nerveux d'un mammifère, ou dans le diagnostic ou le suivi de l'évolution d'une telle maladie autoimmune chez un patient sous traitement afin de déterminer chez ce patient l'efficacité dudit traitement.
Un objet particulier de l'invention concerne ainsi un kit pour cribler des cibles d'intérêt impliquées dans la survenue d'une maladie d'intérêt, par exemple d'une maladie autoimmune. Un autre objet particulier de l'invention concerne par ailleurs un kit pour diagnostiquer une maladie, typiquement une maladie autoimmune telle que décrite précédemment, chez un animal, typiquement un mammifère, de préférence un être humain.

### PREPARATION DE LA PUCE

Un autre objet de l'invention concerne un procédé de préparation d'une puce telle que décrite précédemment, comprenant les étapes suivantes a) de clonage de l'ADNc ou des ADNc d'intérêt ; b) d'expression de la ou des protéines, typiquement de la ou des protéines membranaires, codées par ledit ou lesdits ADNc dans un système de production de protéines, typiquement dans des cellules en culture ; c) de solubilisation des protéines exprimées à l'aide d'un détergent non dénaturant, typiquement un détergent non ionique (aussi identifié en tant que « détergent doux »), permettant la solubilisation desdites protéines tout en préservant leur conformation (structure tridimensionnelle) et leur capacité de fixation à un support ; et d) de dépôt desdites protéines solubilisées sur un support afin d'obtenir la puce d'intérêt.

L'invention concerne également une puce susceptible d'être obtenue à l'aide d'un procédé selon l'invention tel que décrit précédemment.

De manière préférée, la puce selon l'invention permet la détection, par l'homme du métier, des protéines d'intérêt qui se sont fixées sur le support, à l'aide d'une méthode de détection classique, impliquant typiquement un anticorps de détection unique. Une telle puce permet à son fabricant ou à son utilisateur de s'assurer de la bonne expression de la ou des protéines d'intérêt sur ladite puce.

Un moyen d'obtenir une telle puce consiste à cloner la protéine d'intérêt (étape a) de la méthode décrite précédemment) à l'aide d'un vecteur d'expression permettant i) l'insertion d'une étiquette (séquence « *tag* »), telle que décrite précédemment, dans le même cadre de lecture que la séquence codant la protéine d'intérêt, et ii) l'expression d'une protéine fusionnée, de préférence en position C-terminale, à ladite étiquette. Une telle étiquette permet de vérifier et de mesurer l'expression de la protéine d'intérêt qui lui est associée.
Le vecteur d'expression utilisé est de préférence un vecteur directionnel permettant l'insertion du gène d'intérêt suite à son amplification par PCR, sans étape préalable de digestion enzymatique. Un exemple de vecteur directionnel est fourni par la partie expérimentale de la présente demande qui met en oeuvre le vecteur pcDNA6.2/V5/GW/D-TOPO (Invitrogene). Avantageusement, le vecteur utilisé bénéficie en outre de la technologie Gateway qui permet d'envisager le transfert rapide du gène codant la protéine d'intérêt, d'un premier vecteur dans un second vecteur bénéficiant de la même technologie mais ayant des propriétés d'expression différentes (le gène étant cloné dans le vecteur donneur entre deux sites de recombinaisons compatibles avec tous les vecteurs receveurs Gateway). Cette technologie permet par exemple de transférer le gène codant la protéine d'intérêt depuis un vecteur permettant l'expression dans les cellules de mammifères (vecteur donneur) vers un vecteur (vecteur receveur) permettant l'expression dans les cellules d'insectes, les cellules de batracien, les levures, ou les bactéries, ou encore vers un vecteur identique mais dépourvu d'étiquette. Un vecteur bénéficiant de la technologie Gateway peut être choisi par exemple parmi les vecteurs pT-REx™-DEST30 (permettant l'expression dans des cellules de mammifère), pET™-DEST42 (permettant l'expression dans des bactéries), pDEST™8 Vector (permettant l'expression dans des cellules d'insecte), et pYES2-DEST52 (permettant l'expression dans les levures).

Lors de l'amplification des gènes par PCR, l'amorce sens (*"forward primer"*) commence de préférence par la séquence CACC (SEQ ID NO :313) (laquelle précède le codon start (ATG) du gène d'intérêt). Dans un autre mode de réalisation préféré, un nucléotide guanidine (G) est inséré après le codon « start » ATG afin d'obtenir une séquence Kozak. Dans encore un autre mode de réalisation, un codon supplémentaire GGA est introduit dans la séquence nucléotidique à la suite du codon start du gène d'intérêt.
Selon encore un autre mode de réalisation préféré, l'amorce anti-sens (*"reverse primer"*) ne contient pas de codon stop et/ou ne se termine pas par la séquence complémentaire du début de l'amorce sens.
Préférentiellement, les amorces (sens et/ou antisens) présentent une température d'hybridation comprise entre environ 70°C et environ 80°C, ainsi que avantageusement un pourcentage de motifs GC inférieur à environ 60%.

L'étape b) d'expression de la ou des protéines codées par ledit ou lesdits ADNc d'intérêt est avantageusement réalisée dans des cellules en culture, de préférence dans des cellules humaines. Les cellules sont généralement mises en culture sur des plaques multi-puits, traitées ou non pour favoriser l'adhésion des cellules à l'aide de techniques connues de l'homme du métier, chaque puits contenant de préférence des cellules exprimant un seul type de protéine d'intérêt. L'expression de la protéine d'intérêt peut être contrôlée dès ce stade, et si besoin normalisée, grâce à la détection possible (par exemple par immunofluorescence) de la séquence « tag » qui s'exprime, le cas échéant, en même temps que la protéine d'intérêt. Les cellules sélectionnées sont de préférence des cellules présentant des propriétés satisfaisantes d'adhésion au support de culture, qui sont simples à cultiver et à manipuler. De telles cellules peuvent être avantageusement sélectionnées par exemple parmi les lignées HEK293A, COS, MDCK, CHO, CCL39.

La méthode selon l'invention comprend par ailleurs une étape c) de solubilisation de la ou des protéines d'intérêt à l'aide d'un détergeant, de préférence à l'aide d'un détergeant non dénaturant capable de préserver la conformation de la protéine native ainsi que sa capacité de fixation à un support, typiquement à un support tel que décrit précédemment, de préférence à un support solide de type membrane. Les inventeurs ont découvert que le détergeant peut être avantageusement sélectionné parmi le triton X_100, le triton X_114, le Nonidet P-40, le CHAPS, le sodium cholate, le sodium deoxycholate, la digitonine, le sarkosyl NL30 et l'octylglucoside.

La méthode comprend enfin une étape d) de dépôt sur un support tel que décrit précédemment des protéines d'intérêt solubilisées afin d'obtenir la puce selon l'invention. Cette étape est typiquement réalisée par dépôt, sous la forme de spots, des solubilisats obtenus à l'issue de l'étape c) sur le support choisi, à l'aide de techniques connues de l'homme du métier.

### UTILISATIONS

Il n'existe en particulier à ce jour aucun outil permettant de détecter de manière simple et efficace la présence d'autoanticorps dirigés contre des protéines membranaires et/ou complexes protéiques membranaires, dans un échantillon biologique sans devoir procéder à la visualisation par microscopie des complexes antigènes/anticorps (Ag/Ac) formés à la surface de cellules (préalablement transfectées à l'aide de la séquence codant l'antigène d'intérêt) ou de coupes de tissus. La puce selon l'invention est ainsi le premier outil disponible qui permette la détection de complexes Ag de protéine ou complexe protéique membranaire/Ac directement sur un support solide (test sur membrane par exemple).

La puce selon l'invention peut être avantageusement utilisée dans le cadre d'une méthode de diagnostic et/ou de suivi de l'évolution d'une maladie, typiquement d'une maladie autoimmune telle que décrite précédemment, de préférence d'une encéphalopathie associée à des troubles de l'excitabilité neuronale, liée à l'expression de l'une ou de plusieurs des protéines, en particulier des protéines membranaires, d'intérêt fixées sur le support de ladite puce, ou encore dans le cadre d'une méthode de détermination de l'efficacité d'un traitement appliqué à un patient donné. La disparition immédiate ou progressive des autoanticorps recherchés dans l'échantillon biologique provenant d'un patient sous traitement thérapeutique, évaluée à l'aide de la puce à protéines selon l'invention, permet ainsi de vérifier, dans le temps, l'efficacité du traitement appliqué audit patient. L'absence d'effet dudit traitement, facilement détectable à l'aide de ladite puce, permet par ailleurs désormais de corriger ou d'adapter à un stade précoce le traitement appliqué à un patient donné.

La puce selon l'invention peut également être utilisée dans le cadre des méthodes nécessaires de recherche et d'identification (méthodes de criblage) d'anticorps d'intérêt, même présents à très faibles concentrations (i.e. de l'ordre de la millimole), en particulier d'autoanticorps, ainsi que de nouvelles cibles antigéniques de maladies, par exemple de maladies auto-immunes (antigènes d'intérêt impliqués dans la survenue d'une maladie immune).
Les échantillons biologiques de sujets identifiés comme souffrant de pathologies suspectées d'appartenir ou clairement identifiées comme appartenant à la catégorie des maladies auto-immunes peuvent avantageusement être utilisés à cette fin.
Il est ainsi désormais possible de produire et d'utiliser une puce selon l'invention [typiquement une puce qui présente i) l'ensemble des protéines, en particulier des protéines ou complexes protéiques membranaires, exprimées par un tissu donné, ii) l'ensemble des protéines, en particulier des protéines ou complexes protéiques membranaires, impliquées dans un mécanisme physiologique particulier, ou iii) l'ensemble des protéines, en particulier des protéines ou complexes protéiques membranaires, impliquées dans une voie métabolique spécifique] afin de détecter de nouveaux autoanticorps ou d'identifier de nouvelles cibles d'intérêt thérapeutique.

Le criblage à grande ampleur autorisé par ce nouvel outil permet la mise en évidence simultanée d'un très grand nombre de complexes Ag/Ac. Il permet également un archivage des résultats plus simple que celui permis par les tests actuellement connus mis en oeuvre sur des cellules ou des coupes de tissus. Les membranes représentent des surfaces d'environ 4 cm sur 4 cm de l'épaisseur d'une feuille de papier. Elles sont détectées sur une « photo » de taille identique qui peut-être stockée comme toute photo papier ou numérisé. Dans le cas des coupes de tissus ou des cellules transfectées, l'archivage des lames se fait en congélateur.

L'identification facilitée des anticorps, par exemple des autoanticorps impliqués dans des pathologies auto-immunes du système nerveux, et de leurs cibles, à l'aide d'une puce selon l'invention offrant la sensibilité suffisante, permet à présent le développement de tests de diagnostic, en particulier de tests sérologiques plus efficaces, plus simples et plus économiques.

La puce à protéines selon l'invention, grâce aux nouveaux complexes antigènes/anticorps qu'elle permet d'identifier, permet par ailleurs la mise au point de nouveaux traitements thérapeutiques impliquant l'utilisation des anticorps identifiés, ou de modulateurs de la formation desdits complexes antigènes/anticorps, en tant que médicaments, ou dans le cadre de la préparation de compositions pharmaceutiques d'intérêt.

Les figures et exemples suivants illustrent l'invention en décrivant l'obtention de puces conformes à l'invention et en fournissant des exemples d'utilisations desdites puces, sans en limiter la portée.

### LEGENDES DES FIGURES

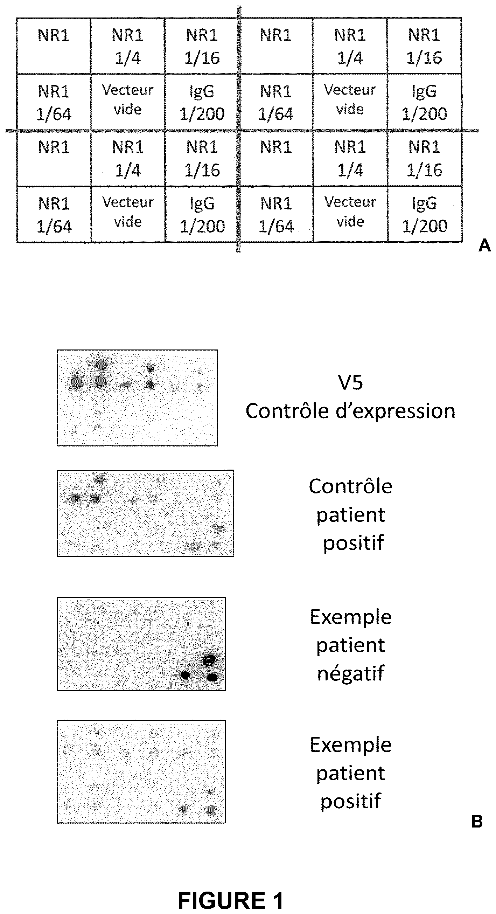

**Figure 1** : Validation de l'efficacité de la puce à protéine grâce à un test pratiqué sur les échantillons provenant de 43 patients.
   **A.** Plan de plaque représentant 4 exemplaires d'une membrane de nitrocellulose sur laquelle sont déposés, pour chaque exemplaire, des concentrations décroissantes d'un extrait de membranes solubilisées de cellules ayant surexprimé le récepteur NR1, un extrait de membranes solubilisées de cellules sans vecteur, et une dilution au 1/200^{ème} de sérum humain.
   **B.** Photos de supports correspondant respectivement à i) un contrôle d'expression à l'aide de l'étiquette V5 seule, ii) un contrôle d'expression à l'aide d'un échantillon provenant d'un patient positif, iii) un exemple d'échantillon négatif, et iv) un exemple d'échantillon positif. Sur les 43 échantillons testés, 15 échantillons sur 17 échantillons positifs ont été détectés à l'aide de la puce à protéines soit une spécificité de la puce à protéines à 88%. 100% des échantillons négatifs ont par ailleurs été détectés.

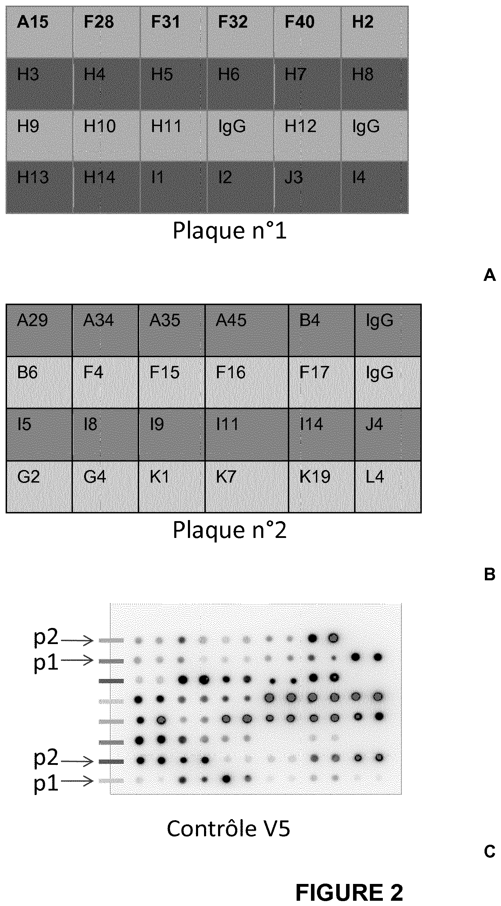

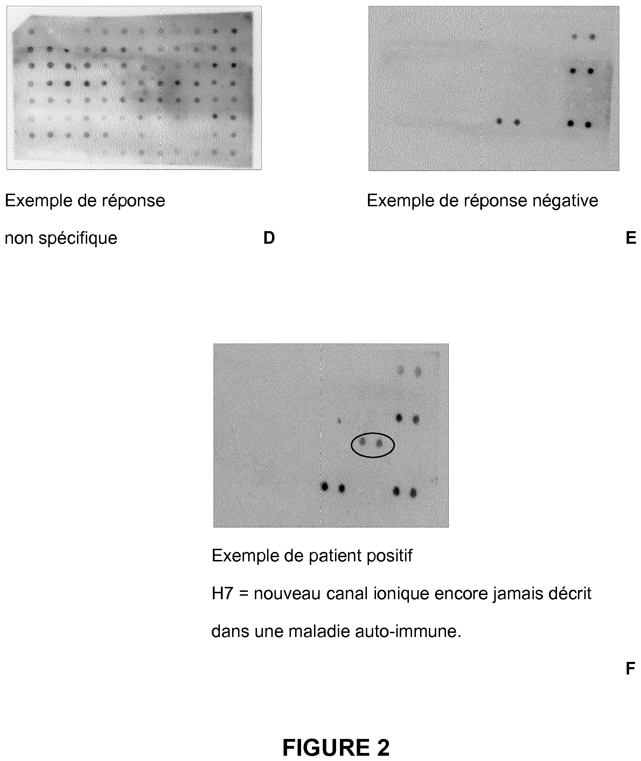

**Figure 2** : Exemple d'utilisation d'une puce à protéines selon l'invention comprenant 40 protéines d'intérêt.
   **A.** Plaque 1: elle contient deux puits contenant des IgG humains et 22 puits contenant les protéines solubilisées de membranes de cellules HEK, chaque cellule exprimant une protéine membranaire différente choisie parmi les protéines identifiées dans le tableau 2 (référencées SEQ ID NO :1 à SEQ ID NO :311).
   **B.** Plaque 2: elle contient deux puits contenant des IgG humains et 22 puits contenant les protéines solubilisées de membranes de cellules HEK, chaque cellule exprimant une protéine membranaire différente choisie parmi les protéines identifiées dans le tableau 2 (référencées SEQ ID NO :1 à SEQ ID NO :311) et non exprimées par les cellules utilisées pour préparer la plaque 1.
   **C.** Plaque contrôle (l'expression correcte est vérifiée à l'aide de l'étiquette V5 seule)
   **D.** Exemple de réponse non spécifique
   **E.** Exemple de réponse négative
   **F.** Exemple de réponse positive

### EXEMPLES

### EXEMPLE 1 - Préparation d'une puce à protéines selon l'invention et révélation des protéines fixées

### Matériels et méthode

### Clonage

Les inventeurs ont utilisé le vecteur d'expression pcDNA6.2-TOPO/GW/V5 (Invitrogène Cat#K2460-20). La majorité des amorces a été commandée chez Eurogentech et le reste chez Sigma.

La PCR est réalisée sur une machine Thermocycler (Biometra) avec un mélange commercial de polymérases (HF enzym mix de Fermentas cat#K0192). Le protocole d'amplification est de type 2-étapes avec une température d'élongation et d'amplification à 68 °C.
La matrice pour l'amplification des gènes d'intérêt est principalement issue d'une banque d'ADNc réalisée à partir d'ARNm de cerveau humain sain (Invitrogen). La transcription reverse de cet ARNm en ADNc est réalisée grâce à une Superscript III (Invitrogen) suivant le protocole du fabricant.
En cas d'échec d'amplification du gène d'intérêt (soit à cause du peu d'expression de ce gène dans le cerveau et donc de la difficulté à l'amplifier à partir de cette banque d'ADNc, soit à cause de la nature nucléotidique de ce gène, soit encore pour une autre raison indéterminée), on utilise une autre source d'ARN (cellule humaines de type HEK par exemple) ou on utilise une construction plasmidique lorsqu'elle est disponible.
Les gènes amplifiés par PCR sont séparés des dNTP et de la matrice par électrophorèse sur gel d'agarose (1%). Les bandes d'ADN de poids moléculaires correspondants aux gènes d'intérêt sont découpés sur gel et purifiés sur colonne (type "gel extraction purification" de QuiAgen, Promega ou Macherey Nagel). Les gènes amplifiés ainsi purifiés sont alors insérés dans le vecteur d'expression selon les directives du fabricant (TOPO-cloning Invitrogen).
Les constructions plasmidiques ainsi créées sont criblées par transformation bactérienne. Etalées sur boites de culture bactérienne, elles sont sélectionnées avec un antibiotique de type Ampicilline. Les bactéries (de type TOP10 Invitrogen) positives sont mises en culture liquide le jour suivant avec la même sélection antibiotique. Cette amplification de culture bactérienne permet l'amplification de la quantité d'ADN disponible. Cet ADN amplifié est purifié sur colonne (de type miniperp Qiagen). Les plasmides susceptibles d'avoir incorporé le gène d'intérêt sont séquencés à l'aide d'une plateforme de séquençage. Les amorces de séquençage sont le T7 forward et un primer reverse spécifique du vecteur pcDNA6.2-TOPO/GW/V5. Seuls le début et la fin de la séquence du gène sont vérifiés par séquençage. En effet, la détection ultérieure de la présence de l'étiquette (ta-V5g) dans les cellules en culture attestera à elle seule de l'intégrité de la protéine.

### Culture cellulaire

Les inventeurs ont utilisé comme système de production protéique la souche de cellules humaine HEK293A (Invitrogene Cat# R70507).
Pour la transfection cellulaire, les inventeurs ont utilisé comme agent transfectant du JetPEI (Ozyme cat#101-10).

Les inventeurs ont transfecté une première série de cellules sur des lamelles de verre préalablement recouvert de poly-L-Lysine afin de faciliter leur adhésion.
Deux jours après la transfection, les inventeurs ont contrôlé par immuno fluorescence la présence du Tag-V5. L'anticorps primaire dirigé contre le *tag* V5 est un anti-V5 de lapin (Anticorps polyclonal Sté Covance Cat#PRB189P). L'anticorps secondaire anti-lapin est couplé à un fluorophore de type Alexa488 ou Alexa594 (Invitrogen). Cette détection fini de valider le clonage du gène d'intérêt.
Une deuxième série de culture cellulaire sur boite de diamètre 60mm, avec le même agent transfectant (Jet-PEI), permet de produire une quantité plus importante de cellules. Deux jours après la transfection de la construction contenant le gène d'intérêt, on récupère les cellules de la façon suivante :
On enlève le milieu de culture,
On rince les cellules avec 1 ml de PBS que l'on aspire aussitôt.
On rince à nouveau avec 1 ml de PBS.
On laisse 10 minutes à température ambiante (RT).
Toute la procédure suivante est avantageusement réalisée dans la glace.
Avec une micropipette (P1000 + pointes à filtres) on décolle les cellules par projection répétée et délicate du PBS contenu dans la boite.
Les cellules resuspendues sont transférées dans un tube de centrifugation (type ependorf) de 1.5 ml.
Les cellules sont centrifugées 5 minutes à 14K RPM (révolution par minute) et à 4°C dans une centrifugeuse de paillasse.
On retire le surnagent.
On re-suspend les cellules dans 50 µl de tampon de solubilisation préparé par exemple comme suit :
40% Glycérol, 2% CHAPS, 140 mM NaCl, 2 mM EDTA, 20 mM Tris pH8.8, dans du PBS additionné d'un mélange d'anti-protéases de type "Complète" (Société Roche).
Les cellules sont solubilisées dans ce tampon par environ 50 va et vient de pipetage. Toujours dans le tube microfuge, le solubilisat est laissé dans la glace pendant une heure.
On centrifuge 10 minutes à 2500 RPM (révolution par minute), et à 4°C.
Le surnageant est transféré dans un tube propre et additionné de tampon de charge pour ADN (loading buffer) puis stocké à -80°C.

Une fois qu'on a établi la collection de plusieurs solubilisats de différentes cultures et de différentes protéines exprimées, on répartit ces protéines solubles dans des boites de 96 puits à fond coniques en utilisant avantageusement les 24 puits centraux.

En maintenant ces boites sur glace, on prélève une fraction de chaque solubilisat grâce à un réplicateur de type « multiwell replicator » et on réalise une empreinte en 3 exemplaires (triplicate) sur une membrane de nitrocellulose (Hybond-C Exra Cat#RPM303C, Amersham). Cette membrane constitue le support pour les 24 solubilisats à tester avec le prélèvement d'un patient. Une empreinte identique est réalisée pour vérifier l'expression correcte des protéines tagguée-V5 dans chaque solubilisat.
Le jour du test on découpe les membranes imprimées à la limite de taille des 24 puits. (On entaille un coin pour orienter la membrane).
On roule cette membrane et on l'introduit dans un tube à hémolyse de 5 ml.
On s'assure que toute la surface de la membrane soit répartie de façon homogène à l'intérieur du tube.
On incube 15 minutes cette membrane avec 2 ml de solution de blocage (PBS, 5 % lait, 0.2% NP40).
On remplace ce milieu de blocage par 750 µl à 1 ml de solution d'anticorps à 1/20^{ème} (50 µl de LCR dans 1 ml de solution de blocage). On incube toute la nuit à température ambiante sur une machine rotative.
On récupère le milieu d'incubation que l'on congèle pour une deuxième utilisation.
On rince 3 fois chaque membrane dans les tubes à hémolyse avec la solution de rinçage (PBS, 0.1% NP40).
On incube 2 heures à température ambiante avec l'anticorps secondaire (dilué au 1/5000^{ème} dans la solution de blocage (PBS, 5 % lait, 0.2% NP40). L'anticorps secondaire est couplé à HRP (HorseRadish Peroxidase) (anti-humain et anti-lapin de Jackson Immunoresearch).
On rince 3 fois avec la solution de rinçage (PBS, 0.1% NP40) et 1 fois avec PBS seul.
On révèle les blots avec 500 µl d'ECL (Enhanced chemoluminescence) (Pierce) dans le révélateur (FujiFilm Fluorescent Image Analyzer FLA-3000).

### EXEMPLE 2 - Validation de l'efficacité de la puce à protéines

Dans le présent exemple, des prélèvements (de type LCR) provenant d'une cohorte de patients connus pour présenter des anticorps dirigés contre la sous-unité NR1 des récepteurs NMDA (initialement détectés par une méthode d'immunocytologie) ainsi que des prélèvements provenant d'individus ne souffrant pas d'une maladie autoimmune sont utilisés pour valider l'efficacité de la puce à protéines selon l'invention.

L'invention à été validée grâce à un test en aveugle réalisé sur différents prélèvements provenant de 17 patients connus pour contenir des anticorps anti-NR1 (au sein d'une population constituée de 26 individus sains).
Selon le protocole décrit plus haut, les protéines membranaires de cellules exprimant le récepteur NR1 sont solubilisées et déposées sur une membrane de nitrocellulose. Chaque membrane présente 4 dépôts de la protéine d'intérêt correspondant à 4 dilutions décroissantes de la même préparation de protéines membranaires solubilisées issue de cellules sur-exprimant la sous-unité NR1, un dépôt contrôle correspondant à des protéines membranaires solubilisées à partir de cellules exprimant le vecteur vide, un dépôt contrôle correspondant à une dilution de sérum humain issu d'un sujet sain (voir figure 1). Après incubation et détection comme décrit plus haut, les résultats ont montré que sur les 17 patients malades, le test en a reconnu 15 et présente donc un efficacité de 88%. La spécificité pour les patients négatif est de 100%. Les 2 faux positifs reconnus par la puce à protéines selon l'invention correspondent à deux candidats qui avaient été détectés positifs puis écartés en deuxième criblage d'un premier test pratiqué sur cellules.

### EXEMPLE 3 - Criblage mettant en oeuvre la puce à protéines

Dans le présent exemple, l'invention a été testée sur une cohorte de 50 patients souffrant de troubles neurologiques d'origine inconnue (et potentiellement auto-immuns) pour lesquels les inventeurs ont cherché à savoir s'ils possédaient des anticorps dirigés contre une ou plusieurs protéines parmi 40 protéines déposées sur une membrane de nitrocellulose.
Dans cet exemple, chaque plaque (Figure 2) possède 20 échantillons de protéines membranaires solubilisées issues de cellules sur-exprimant individuellement 20 protéines d'intérêt (codifiées ici A15, F28, F31, etc.) ainsi que 2 échantillons correspondant à une dilution de sérum humain issu d'un sujet sain.
Les dépôts sur membrane de nitrocellulose ont été réalisés selon le protocole décrit plus haut (avec un réplicateur) en duplicata et en combinant sur la même membrane les dépôts des deux plaques (plaque 1 en bas, plaque 2 en haut) (voir figure 2).

Les résultats ont montré que parmi les 50 patients testés, de nombreux patients étaient négatifs et donc ne possédaient pas d'anticorps dirigés contre une des 40 protéines déposées, un petit nombre possédaient des anticorps contre toutes les protéines déposées, un patient possédait des anticorps dirigés spécifiquement contre une protéine (H7).
Après confrontation de ces résultats avec le tableau clinique de la cohorte de 50 patients, il apparait que la réponse non spécifique des patients possédant des anticorps dirigés contre toutes les protéines s'explique en partie par le degré d'avancement et de gravité de leur maladie puisque la plupart d'entre eux sont décédés avant même la mise en place du test (réponse immune massive et non spécifique). En revanche, le résultat unique concernant la protéine H7 est le parfait exemple de l'identification possible à l'aide de la puce selon l'invention de l'association (mise en évidence pour la première fois) existant entre un canal particulier (protéine identifiée dans le présent exemple en tant que protéine H7) et une maladie neurologique.

## Revendications

1. Procédé de préparation d'une puce à protéine, comprenant les étapes suivantes :
a) clonage de l'ADNc ou des ADNc d'intérêt codant au moins une protéine membranaire s'exprimant dans les cellules du système nerveux d'un animal, impliquée ou suspectée d'être impliquée dans la survenue d'une encéphalopathie associée à des troubles de l'excitabilité neuronale,
b) expression de la ou des protéines membranaires codées par ledit ou lesdits ADNc dans des cellules en culture, lesdites cellules en culture étant des cellules de mammifères, à l'aide d'un vecteur permettant i) l'insertion d'une étiquette (séquence tag) dans le même cadre de lecture que la protéine d'intérêt et ii) l'expression d'une protéine fusionnée à ladite étiquette,
c) solubilisation des protéines fusionnées à ladite étiquette exprimées à l'aide d'un détergent non dénaturant permettant la solubilisation desdites protéines membranaires tout en préservant leur conformation et leur capacité de fixation à un support, pour obtenir un solubilisat et
d) dépôt du solubilisat desdites protéines membranaires obtenu à l'issue de l'étape c) sur un support et obtention de la puce d'intérêt.

2. Procédé selon la revendication 1, **caractérisé en ce que** le détergent est un détergent choisi parmi le triton X_100, triton X_114, Nonidet P-40, CHAPS, Sodium cholate, Sodium deoxycholate, digitonine, sarkosyl NL30 et l'octylglucoside.

3. Procédé selon l'une quelconque des revendications 1 ou 2, **caractérisé en ce que** ladite protéine membranaire est associée à une ou plusieurs sous- unités auxiliaires.

4. Procédé selon l'une quelconque des revendications précédentes, **caractérisé en ce que** ladite protéine membranaire d'intérêt solubilisée étant choisie parmi une protéine canal ionique, une protéine transporteur, et une protéine récepteur membranaire régulant l'activité d'une protéine canal ionique ou d'une protéine transporteur.

5. Procédé selon l'une quelconque des revendications précédentes, **caractérisé en ce que** ladite au moins une protéine membranaire s'exprime dans les cellules du système nerveux d'un être humain.

6. Procédé selon l'une quelconque des revendications précédentes, **caractérisé en ce que** l'encéphalopathie associée à des troubles de l'excitabilité neuronale est sélectionnée parmi une encéphalite post- infectieuse, l'encéphalite anti-NMDAR, le Syndrome de MORVAN, la maladie de Devic, un syndrome opso-myoclonique de l'enfant, une encéphalite limbique, l'encéphalite de Rasmussen, une encéphalomyélite, un syndrome épileptique acquis et un syndrome neurologique paranéoplasique (SNP).

7. Procédé selon l'une quelconque des revendications précédentes, **caractérisé en ce que** ladite au moins une protéine membranaire consiste en une séquence d'acides aminés choisie parmi SEQ ID NO: 1 à SEQ ID NO : 311.

8. Procédé selon l'une quelconque des revendications précédentes, **caractérisé en ce que** la protéine membranaire d'intérêt est choisie parmi un récepteur du GABA ; un récepteur du glutamate ; l'aquaporine 2 (SEQ ID NO :307) ; l'aquaporine 4 (SEQ ID NO :309) ; un canal potassique ; un canal calcique ; un récepteur de l'acétylcholine, une protéine associée aux canaux potassiques dépendant du voltage; un récepteur Glycine; la connexine 32 (SEQ ID NO :310), et la connexine 43 (SEQ ID NO :311).

9. Procédé selon l'une quelconque des revendications précédentes, **caractérisé en ce que** la protéine membranaire d'intérêt est un récepteur au glutamate activé par la N-méthyl-D-aspartate (NMDA), de préférence le récepteur NMDAR1 (SEQ ID NO : 62).

10. Puce susceptible d'être obtenue à l'aide d'un procédé selon l'une des revendications 1 à 9.

11. Puce selon la revendication 10, **caractérisée en ce qu'**elle comprend au moins 100 protéines membranaires d'intérêt différentes, de préférence entre environ 200 et environ 400 protéines d'intérêt différentes, par exemple environ 250 ou environ 300 protéines d'intérêt différentes, chaque protéine d'intérêt étant éventuellement associée à une ou à plusieurs sous-unités auxiliaires.

12. Puce selon l'une quelconque des revendications 10 ou 11, **caractérisée en ce que** ladite ou lesdites protéines membranaires d'intérêt sont marquées à l'aide d'une même séquence peptidique reconnue par un anticorps de détection.

13. Puce selon l'une quelconque des revendications 10 à 12, **caractérisée en ce qu'**elle comprend un support susceptible de fixer des protéines membranaires d'intérêt solubilisées choisi parmi une membrane, typiquement une membrane de nitrocellulose ou de nylon ; du verre ; et du plastique.

14. Utilisation d'une puce selon l'une des revendications 10 à 13, pour le criblage dans un échantillon biologique choisi parmi le sang, le sérum, le plasma, le liquide céphalo-rachidien (LCR), l'endolymphe de l'oreille interne, la périlymphe de l'oreille interne et une sous-fraction de ceux-ci, d'un auto-anticorps d'intérêt impliqué dans la survenue d'une encéphalopathie associée à des troubles de l'excitabilité neuronale.

15. Utilisation d'une puce selon l'une des revendications 10 à 13, pour le diagnostic ou le suivi de l'évolution d'une encéphalopathie associée à des troubles de l'excitabilité neuronale.

16. Kit comprenant une puce selon l'une des revendications 10 à 13, et éventuellement un ou plusieurs réactifs choisis parmi un tampon de blocage des sites non spécifiques, un tampon permettant l'association entre anticorps et antigènes, un tampon de lavage, un anticorps secondaire, un produit de détection dudit anticorps secondaire, et des instructions d'utilisation.

## Patentansprüche

1. Verfahren zur Herstellung eines Proteinchips, das die folgenden Schritte umfasst:
a) Klonieren einer oder mehrerer cDNA von Interesse, die für mindestens ein Membranprotein codiert/codieren, das in den Zellen den Nervensystems eines Tieres exprimiert wird und das am Auftreten einer Enzephalopathie, die mit Störungen der Erregbarkeit der Neuronen in Verbindung steht, beteiligt ist oder mutmaßlich beteiligt ist,
b) Exprimieren des oder der Membranproteine, die von der/den cDNA codiert werden, in angezüchteten Zellen, wobei es sich bei den angezüchteten Zellen um Säugetierzellen handelt, mit Hilfe eines Vektors, der es ermöglicht, i) einen Marker (Sequenzmarker) in denselben Leserahmen wie das Protein von Interesse einzuführen, und ii) Exprimieren eines Proteins, das mit dem Marker fusioniert ist,
c) Inlösungbringen der exprimierten Proteine, die mit dem Marker fusioniert sind, mit Hilfe eines nichtdenaturierenden Detergens, das es ermöglicht, die Membranprotein in Lösung zu bringen, wobei deren Tertiärstruktur und deren Fähigkeit zur Befestigung an einem Träger erhalten bleiben, um ein Solubilisat zu erhalten, und
d) Aufbringen des Solubilisats der Membranproteine, wie es nach Abschluss des Schrittes c) erhalten wurde, auf einen Träger und Erhalten des Chips von Interesse.

2. Verfahren nach Anspruch 1, **dadurch gekennzeichnet, dass** es sich bei dem Detergens um ein Detergens handelt, das aus Triton X_100, Triton X_114, Nonidet P-40, CHAPS, Natriumcholat, Natriumdesoxycholat, Digitonin, Sarkosyl NL30 und Octylglucosid ausgewählt ist.

3. Verfahren nach einem beliebigen der Ansprüche 1 oder 2, **dadurch gekennzeichnet, dass** das Membranprotein mit einer oder mehreren unterstützenden Untereinheiten in Verbindung steht.

4. Verfahren nach einem beliebigen der vorhergehenden Ansprüche, **dadurch gekennzeichnet, dass** das in Lösung gebrachte Membranprotein von Interesse aus einem Ionenkanalprotein, einem Transporterprotein und einem membranständigen Rezeptorprotein ausgewählt ist, das die Aktivität eines Ionenkanalproteins oder eines Transporterproteins reguliert.

5. Verfahren nach einem beliebigen der vorhergehenden Ansprüche, **dadurch gekennzeichnet, dass** das mindestens eine Membranprotein in den Zellen des Nervensystems eines Menschen exprimiert wird.

6. Verfahren nach einem beliebigen der vorhergehenden Ansprüche, **dadurch gekennzeichnet, dass** die Enzephalopathie, die mit Störungen der Erregbarkeit der Neuronen in Verbindung steht, aus einer postinfektiösen Enzephalitis, der Anti-NMDA-Rezeptor-Enzephalitis, dem Morvan-Syndrom, dem Devic-Syndrom, einem Opso-Myoklonus-Syndrom beim Kind, einer limbischen Enzephalitis, der Rasmussen-Enzephalitis, einer Enzephalomyelitis, einem erworbenen epileptischen Syndrom und einem neurologisch-paraneoplastischen Syndrom ausgewählt ist.

7. Verfahren nach einem beliebigen der vorhergehenden Ansprüche, **dadurch gekennzeichnet, dass** das mindestens eine Membranprotein aus einer Aminosäuresequenz besteht, die aus der SEQ ID Nr.: 1 bis SEQ ID Nr.: 311 ausgewählt ist.

8. Verfahren nach einem beliebigen der vorhergehenden Ansprüche, **dadurch gekennzeichnet, dass** das Membranprotein von Interesse aus einem GABA-Rezeptor; einem Glutamat-Rezeptor; Aquaporin 2 (SEQ ID Nr.: 307); Aquaporin 4 (SEQ ID Nr.: 309); einem Kaliumkanal; einem Calciumkanal; einem Acetylcholin-Rezeptor, einem Protein, das mit spannungsabhängigen Kaliumkanälen in Verbindung steht; einem Glycinrezeptor; Connexin 32 (SEQ ID Nr.: 310) und Connexin 43 (SEQ ID Nr.: 311) ausgewählt ist.

9. Verfahren nach einem beliebigen der vorhergehenden Ansprüche, **dadurch gekennzeichnet, dass** es sich bei dem Membranprotein von Interesse um einen Glutamat-Rezeptor handelt, der durch N-Methyl-D-aspartat (NMDA) aktiviert wird, vorzugsweise um den Rezeptor NMDAR1 (SEQ ID Nr.: 62).

10. Chip, der mit Hilfe eines Verfahrens nach einem der Ansprüche 1 bis 9 erhalten werden kann.

11. Chip nach Anspruch 10, **dadurch gekennzeichnet, dass** er mindestens 100 verschiedenartige Membranproteine von Interesse, vorzugsweise zwischen ungefähr 200 und ungefähr 400 verschiedenartige Proteine von Interesse, beispielsweise ungefähr 250 oder ungefähr 300 verschiedenartige Proteine von Interesse umfasst, wobei jedes Protein von Interesse möglicherweise mit einer oder mehreren unterstützenden Untereinheiten in Verbindung steht.

12. Chip nach einem beliebigen der Ansprüche 10 bis 11, **dadurch gekennzeichnet, dass** das oder die Membranproteine von Interesse mit Hilfe ein und derselben Peptidsequenz markiert sind, die von einem Detektionsantikörper erkannt wird.

13. Chip nach einem beliebigen der Ansprüche 10 bis 12, **dadurch gekennzeichnet, dass** er einen Träger umfasst, auf dem die in Lösung gebrachten Membranproteine von Interesse befestigt werden können, wobei er aus einer Membran, typischerweise einer Nitrocellulose- oder Nylonmembran; Glas; und Kunststoff ausgewählt ist.

14. Verwendung eines Chips nach einem der Ansprüche 10 bis 13, um eine biologische Probe, die aus Blut, Serum, Plasma, Gehirn-Rückenmarksflüssigkeit, Endolymphe des Innenohrs, Perilymphe des Innenohrs sowie einer Teilfraktion davon ausgewählt ist, auf einen Autoantikörper von Interesse zu screenen, der am Auftreten einer Enzephalopathie beteiligt ist, die mit Störungen der Erregbarkeit der Neuronen in Verbindung steht.

15. Verwendung eines Chips nach einem der Ansprüche 10 bis 13 zur Diagnose oder Beobachtung des Verlaufs einer Enzephalopathie, die mit Störungen der Erregbarkeit der Neuronen in Verbindung steht.

16. Kit, das einen Chip nach einem der Ansprüche 10 bis 13 sowie möglicherweise ein oder mehrere Reagenzien, die aus einem Puffer zum Blockieren unspezifischer Stellen, einem Puffer, der es ermöglich, die Verbindung zwischen Antikörpern und Antigenen herzustellen, einem Waschpuffer, einem sekundären Antikörper, einem Mittel zur Detektion des sekundären Antikörpers ausgewählt sind, und Gebrauchsanweisungen umfasst.

## Claims

1. A method for preparing a protein chip comprising the following steps:
a) cloning cDNA or cDNAs of interest encoding at least one membrane protein that is expressed in the cells of the nervous system of an animal, involved or suspected of being involved in the onset of encephalopathy associated with neuronal excitability disorders;
b) expressing the one or more membrane proteins encoded by said one or more cDNA into cultured cells, said cultured cells being mammalian cells, using a vector for i) inserting a tag (tag sequence) into the same reading frame as the protein of interest and ii) expressing a protein fused to said tag;
c) solubilizing proteins fused to said tag expressed using a non-denaturing detergent for solubilizing said membrane proteins while maintaining their conformation and their capacity for binding to a support, to obtain a solubilizate; and
d) depositing the solubilizate of said membrane proteins obtained after step c) onto a support and obtaining the chip of interest.

2. The method as claimed in claim 1, **characterized in that** the detergent is a detergent selected from triton X_100, triton X_114, Nonidet P-40, CHAPS, sodium cholate, sodium deoxycholate, digitonine, sarkosyl NL30 and octylglucoside.

3. The method as claimed in any one of claims 1 or 2, **characterized in that** said membrane protein is associated with one or more auxiliary sub-units.

4. The method as claimed in any one of the preceding claims, **characterized in that** said solubilized membrane protein of interest is selected from an ion channel protein, a carrier protein, and a membrane receptor protein that regulates the activity of an ion channel protein or of a carrier protein.

5. The method as claimed in any one of the preceding claims, **characterized in that** said at least one membrane protein is expressed in the cells of the nervous system of a human being.

6. The method as claimed in any one of the preceding claims, **characterized in that** encephalopathy associated with neuronal excitability disorders is selected from post-infectious encephalitis, anti-NMDAR encephalitis, MORVAN syndrome, Devic's disease, opsoclonus-myoclonus syndrome of a child, limbic encephalitis, Rasmussen encephalitis, encephalomyelitis, acquired epileptic syndrome and paraneoplastic neurological syndrome (PNS).

7. The method as claimed in any one of the preceding claims, **characterized in that** said at least one membrane protein consists of an amino acid sequence selected from SEQ ID NO: 1 to SEQ ID NO: 311.

8. The method as claimed in any one of the preceding claims, **characterized in that** the membrane protein of interest is selected from a GABA receptor; a glutamate receptor; aquaporin 2 (SEQ ID NO: 307); aquaporin 4 (SEQ ID NO: 309); a potassium channel; a calcium channel; an acetylcholine receptor, a protein associated with voltage-dependent potassium channels; a glycine receptor; connexin 32 (SEQ ID NO: 310), and connexin 43 (SEQ ID NO: 311).

9. The method as claimed in any one of the preceding claims, **characterized in that** the membrane protein of interest is an N-methyl-D-aspartate (NMDA) activated glutamate receptor, preferably the NMDAR1 receptor (SEQ ID NO: 62).

10. A chip that can be obtained by means of a method according to any one of claims 1 to 9.

11. The chip as claimed in claim 10, **characterized in that** it comprises at least 100 different membrane proteins of interest, preferably between approximately 200 and approximately 400 different proteins of interest, for example, approximately 250 or approximately 300 different proteins of interest, each protein of interest optionally being associated with one or more auxiliary sub-units.

12. The chip as claimed in any one of claims 10 or 11, **characterized in that** said one or more membrane proteins of interest is/are labeled using the same peptide sequence recognized by a detection antibody.

13. The chip as claimed in any one of claims 10 to 12, **characterized in that** it comprises a support capable of fixing solubilized membrane proteins of interest selected from a membrane, typically a membrane of nitrocellulose or of nylon, glass, and plastic.

14. Use of a chip as claimed in any one of claims 10 to 13, for screening in a biological sample selected from blood, serum, plasma, cerebrospinal fluid (CSF), endolymph of the inner ear, perilymph of the inner ear and a sub-fraction thereof, of an auto-antibody of interest involved in the onset of encephalopathy associated with neuronal excitability disorders.

15. The use of a chip as claimed in any one of claims 10 to 13 for diagnosing or monitoring the development of encephalopathy associated with neuronal excitability disorders.

16. A kit comprising a chip as claimed in any one of claims 10 to 13, and optionally one or more reagents selected from non-specific sites blocking buffer, a buffer for the association between antibodies and antigens, a wash buffer, a secondary antibody, a product for detecting said secondary antibody, and instructions for use.
